# EUROPEAN PATENT SPECIFICATION

(11) **EP 2 988 590 B1**
(45) Date of publication and mention of the grant of the patent: **12.08.2020**
(21) Application number: 14788964.6
(22) Date of filing: 24.04.2014
(51) Int. Cl.: A01H 5/00, C12N 15/82, C07K 14/415

(54) **PLANTS RESISTANT TO PATHOGENIC MICROORGANISMS GROWING IN VASCULAR TISSUES**
GEGEN IN VASKULÄREM GEWEBE WACHSENDE PATHOGENE MIKROORGANISMEN BESTÄNDIGE PFLANZEN
PLANTES RÉSISTANTES À DES MICRO-ORGANISMES PATHOGÈNES EN CROISSANCE DANS DES TISSUS VASCULAIRES

(30) Priority: 24.04.2013 MX 2013004623
(43) Date of publication of application: 02.03.2016
(73) Proprietor: Centro de Investigacion y de Estudios Avanzados del Instituto Politécnico Nacional, Ciudad de Mexico 07360 (MX); Secretaría de Agricultura, Ganadería, Desarrollo Rural, Pesca y Alimentacion, México D.F. 03300 (MX)
(72) Inventor: XOCONOSTLE CÁZARES, Beatriz, México, D.F., 07360 (MX); CHAVARIN PALACIO, Claudio, México, D.F., 03300 (MX); LÓPEZ BUENFIL, José Abel, México, D.F., 03300 (MX); GUERRA LUPIÁN, Miguel Ángel, México, D.F., 07360 (MX); MORALES GALVÁN, Oscar, México, D.F., 03300 (MX); ZECUA NÁJERA, Rebeca, México, D.F., 07360 (MX); RUÍZ MEDRANO, Roberto, México, D.F., 07360 (MX); GÓMEZ FELIPE, Andrea, México, D.F., 07360 (MX); TRUJILLO ARRIAGA, Francisco Javier, México, D.F., 04100 (MX)
(74) Representative: Predazzi, Valentina
(86) International application number: PCT/IB2014/060965
(87) International publication number: WO 2014/174474

(56) References cited:
- EP-A2- 0 497 366
- WO-A1-2009/064255
- WO-A2-99/66055
- WO-A2-03/014150
- JP-A- 2000 262 169
- JP-A- 2000 262 169
- DATABASE EMBL [Online] 20 January 1999 (1999-01-20), "Cucurbita maxima phloem protein (PP16) mRNA, PP16-1 allele, complete cds.", XP002761918, retrieved from EBI accession no. EM_STD:AF079170 Database accession no. AF079170
- XOCONOSTLE-CAZARES BEATRIZ ET AL: "Plant paralog to viral movement protein that potentiates transport of mRNA into the phloem", SCIENCE, AMERICAN ASSOCIATION FOR THE ADVANCEMENT OF SCIENCE, US, vol. 283, no. 5398, 1 January 1999 (1999-01-01), pages 94-98, XP002148593, ISSN: 0036-8075, DOI: 10.1126/SCIENCE.283.5398.94
- ROBERTS A G ET AL: "Plasmodesmata and the control of symplastic transport", PLANT CELL AND ENVIRONMENT, WILEY-BLACKWELL PUBLISHING LTD, GB, vol. 26, no. 1, 1 January 2003 (2003-01-01), pages 103-124, XP002405945, ISSN: 0140-7791, DOI: 10.1046/J.1365-3040.2003.00950.X
- XOCONOSTLE-CAZARES BEATRIZ ET AL.: 'Plant paralog to viral movement protein that potentiates transport of mRNA into the phloem' SCIENCE . AMERICAN ASSOCIATION FOR THE ADVANCEMENT OF SCIENCE vol. 283, 1999, pages 94 - 98, XP002148593

## Description

### Field of the invention.

The present invention relates to the field of application of molecular biology techniques to generate plants resistant to phytopathogenic microorganisms residing in the vascular tissue of the plants through the expression of proteins with antimicrobial activity translationally fused to proteins capable of supracellular and systemic movement. In one of its embodiments, the invention relates to methods for controlling the bacteria causing the citrus yellowing or Huanglongbing (HLB) by constitutive expression and tissue-specific vascular antimicrobial proteins fused to proteins capable of simplasmic movement to distant tissues through the vascular tissue.

### Background of the invention.

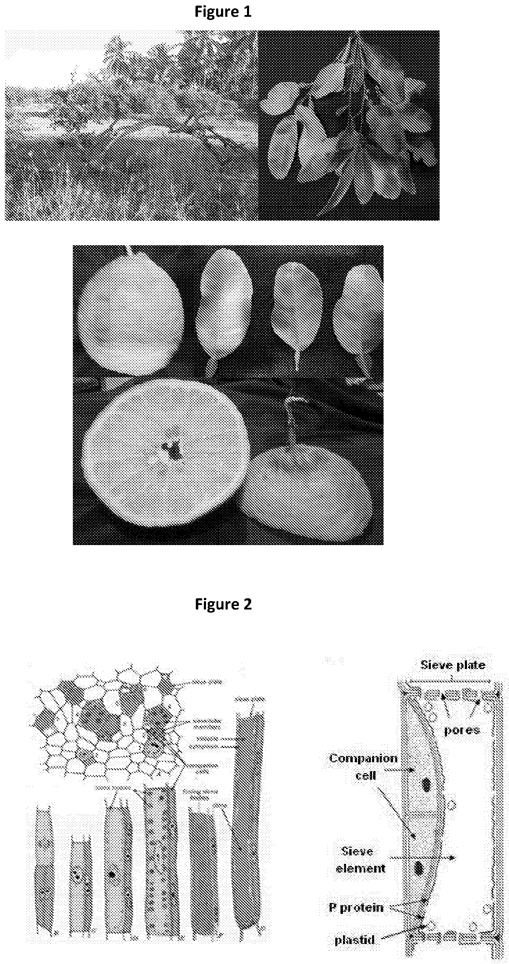

Huanglongbing ("yellow dragon" in Chinese) is a disease of citrus that has taken on great importance in Mexico. It is also known by the initials HLB and the English word Greening or Ex-Greening. The disease is very destructive, because it causes total losses in citrus production. HLB symptoms include but are not restricted to leaf yellowing, reduced production of fruits and practically, the absence of seeds (figure 1). Moreover and coincidentally with the vast majority of diseases in plants of agricultural interest, there is no effective treatment for HLB. For this reason, early diagnosis is crucial and a treatment would be essential to reverse the disease. Note that the disease has spread rapidly around the world since its detection in East Asia; it has now been detected in Florida and Mexico. It is also considered as the main threat to citrus production worldwide (www.senasica.sagarpa.gob.mx).

The HLB disease was first reported in China in 1929, which spread rapidly and was detected in South Africa in 1947; it has also been a severe problem in Taiwan's citrus production since 1951. Thus, it has scattered mainly in citrus growing regions both tropical and subtropical, including Florida, USA, since 1998. In the case of Mexico, it has been reported in several producing regions of the country since 2009, from Sinaloa to Campeche. It is likely that the disease is already present in other citrus producing regions in Mexico, making its control a priority.

In Mexico, there are 23 citrus-producing states, in 13 of which the HLB bacterium and the insect vector transmitting the disease have already been detected. This phytosanitary problem is crucial since citrus is grown on 549,000 hectares with a production of seven million tons annually, valued at $10.206 billion Mexican pesos (SENASICA, 2011).

Among the citrus that are susceptible to HLB infection, the more severe symptoms occur in the Mexican lime, orange *(Citrus sinensis),* Mandarin orange *(Citrus reticulata),* and grapefruits *(Citrus reticulata x Citrus paradisi),* while less severe symptoms are present in limes (*Citrus limon*) and tangerines (*Citrus paradisi*), *Citrus limonia, Citrus limettioides* (McClean & Schwarz, cited in EPPO quarantine pest, 1990). However, worldwide the list of infected plants is greater, wherein the susceptible species are listed below (Halbert and Manjunath, 2004): *Aeglopsis chevalieri Swingle, Atalantia missionis Oliver, Balsamocitrus dawei Stapf., Calodendrum capensis Thunb, Catharanthus roseus (L.) G. Don X Citroncirus webberi J. Ingram & H.E. Moore, Citrus amblycarpa Ochse, Citrus aurantiifolia (Christm.) Swingle, Citrus aurantium L., Citrus depressa Hayata, Citrus grandis (L.) Osbeck, Citrus hassaku Hort. ex Tanaka, Citrus hystrix DC., Citrus ichangensis Swingle, Citrus jambhiri Lushington, Citrus junos Sieb. ex Tanaka, Citrus kabuchi Hort. ex Tanaka, Citrus limon (L.) Burm., Citrus x limonia Osbeck, Citrus x nobilis Lour. "Ortanique", Citrus maxima (pomelo*/*shaddock), Citrus x nobilis Lour., Citrus oto Hort. ex Tanaka, Citrus x paradisi Macfad., Citrus reticulata Blanco, Citrus sinensis (L.) Osbeck, Citrus sunki Hort. ex Tanaka, Citrus unshiu (Mack.) Marc, Clausena indica Oliver, Clausena lansium (Lour.) Skeels, Cuscuta australis R. Br. (Convolvulaceae, Cuscutaceae), Fortunella spp., Limonia acidissima L., Microcitrus australasica (F.J. Muell.) Swingle, Murraya koenigii (L.), Murraya paniculata (L.) Jack, Poncirus trifoliata (L.) Raf., Swinglea glutinosa (Blanco) Merr., Toddalia lanceolata Lam, and Triphasia trifolia (Burm. f.) P*. *Wilson.*

Likewise, the non-hosts include *Citrus indica Tanaka, Citrus limetta Risso, and Citrus macroptera Montrons* (Gomez, 2008).

It has been recognized that the etiology of the disease is bacterial (Liberibacter); however, there are various related strains (table 1). It has been found that HLB is transmitted by insects of the psyllid group (Hemiptera), which includes other important vectors such as, for example, the white midge vectors and aphids. Similar to other vector-borne diseases, one of the strategies used for controlling this disease is precisely the control of the vectors associated with it. While the mechanisms by which psyllid transmit the disease are not precisely known, it is clear that these insects are capable of transmitting it very efficiently. Moreover, psyllids transmit pathogens similarly to aphids, so we can wait that the bacteria transmition details would be similar to other pathogens restricted to the phloem. They are also capable of transmitting the virus, which makes the control of these vectors even more necessary.

One of the vectors of the *Candidatus Liberibacter spp* bacteria causing HLB is *Diaphorina citri* Kuwayama (Hemiptera: Psyllidae); this insect has an embryonic period ranging from 9.7 days at 15°C to 3.5 days at 28°C; the eggs are placed at the end of the tender shoots of the plant, on and between young folded leaves, appearing frequently in large numbers in the same twig; the oviposition is conditional on the presence of tender shoots, where the female puts up to 800 eggs throughout her life, the nymphs are sedentary and settle on the young twigs and petioles, forming colonies with a varying number of individuals. Nymphs excrete a waxy white substance through threads deposited on the leaves, while adults have little ability to hold long flights, but can be transported for long distances by air currents.

**Table 1. Bacteria associated to Huanglongbing (HLB)*.**

| **Agent** | **Host** | **Characteristics** | **Distribution** | **Natural vector** |
|---|---|---|---|---|
| **Liberibacter associated with HLB ^{a}** | | | | |
| ***Candidatus Liberibacter asiaticus*** | *Citrus spp.;* transmitted to *Catharantus roseus,* tobacco *(Nicotiana tabacum),* tomato *(Lycopersicon esculentum)* through parasitic plants (*Cuscuta campestris)* | Gram negative, non cultivable, restricted to vascular tissue, heat tolerant | Mexico, Asia, Saudi Arabia, Florida, Brazil | *Diaphorina citri* (Asiatic citrus psyllid) |

| **Liberibacter of non-rutaceae hosts ^{b}** | | | | |
|---|---|---|---|---|
| ***Candidatus* Liberibacter *psyllarous*** | Solanaceae | 97% similarity in sequence with *Candidatus Liberibacter* cause of "zebra chip" in potatoes | Texas, Guatemala, Mexico | *Bactericera Cockerell* (potato/tomato psyllid) |

| **Phytoplasma associated with HLB** | | | | |
|---|---|---|---|---|
| Phytoplasma ^{c} | Citrus, *Crotolaria juncea* | Restricted to sieved tube, without cell walls, with 99% homology in DNA with the pea witches'-broom phytoplasma (16Sr IX) | Brazil | Unknown |
| Phytoplasma ^{d} | Citrus | | China | Unknown |

| | | | | |
|---|---|---|---|---|
| References: *Strategic Planning for the Florida Citrus Industry: Addressing Citrus Greening; a Bové et al., 1974; Garnier and Bové, 1983; Jagoueix et al., 1994, 1997; Garnier et al., 2000, Duan et al., 2008; ^{b} Gudmestad and Secor, 2007; Hansen et al., 2008; Lin et al., 2009; ^{c} Teixeira et al., 2008; Wulffet et al., 2009; ^{d} Chen et al., 2009 | | | | |

The duration of the biological cycle of the insect (egg-adult) varies from 14.1 to 49.3 days at 28°C and 15°C respectively, wherein temperatures of 25°C to 28°C are the most suitable for its development as the psyllid does not develop at 33°C and 10°C temperatures. The insect has a population peak in late spring and early summer (coinciding with the budding period of citrus). The presence of the psyllid does not imply its infection with the bacteria, although in the case of Mexico, both elements are present.

It has been determined that HLB is caused by a group of bacteria that form a coherent clade, generally called *Candidatus Liberibacter, Candidatus Liberibacter asiaticus, Candidatus Liberibacter africanus,* and *Candidatus Liberibacter americanus.* Mexico has reported that *Candidatus Liberibacter asiaticus* is the bacteria transmitted by *Diaphorina citri* psyllid and is present in the Rutaceae family; however, there are reports of the presence of *Candidatus Liberibacter psyllaurous* whose host plants are the Solanaceae. Brazil and China have reported phytoplasma associated with HLB also restricted to the vascular system of infected plants.

To date it has not been possible to cultivate these bacterial groups, which has prevented further characterization of these pathogens. While the taxonomic relationship of *Candidatus Liberibacter spp.* with other groups is not fully clear, the DNA sequences available indicate that it is an alpha-proteobacteria. Available information suggests that it is related to rhizobacteria such as *Bradyrhizobium,* and more distant with *Agrobacterium.* These bacterial groups have a cell wall composed of peptidoglicane and thus, they could be susceptible to destroy enzymes such as lysozyme.

The *Candidatus Liberibacter* (CaL) bacteria are restricted to the phloem of their host; there are a great variety of plant pathogens, which also accumulate in the phloem, or that are restricted to the tissue, from viruses (such as geminivirus and luteovirus) to bacteria (e.g. principally phytoplasms). The case of fungi has been little studied, but it is possible that many endophytes colonize the phloem. The knowledge of the role of this tissue is essential for developing strategies for these pathogens.

Significantly, Gram-negative bacteria are susceptible to lysozyme in high hydrostatic pressure (for example, a buffer with 10 mM potassium phosphate (Marsschalk et al., 2001)), a condition found in the phloem of the plant. The symptoms of their infection are sometimes confused by nutritional deficiencies, but as the infection progresses the asymmetry of the chlorotic spots present in the disease differs from the symmetric spots on both sides of the central nervures, which are precisely produced by nutritional deficiency (figure 1).

Particularly, the accumulation in the phloem of these pathogens appears to cause most of the observed symptoms. The ultrastructural analysis of infected tissue has shown that the free-flow of phloem is blocked by massive deposits of callose (beta 1-3 glucanase) in sieve plates. The plant organs, which are interconnected through blocked vascular bundles develop chlorosis; this will surely cause a lower growth-rate of the infected plants. Callose accumulation could be an uncontrolled response to the presence of these bacteria in the phloem, which also suggests a treatment against the disease (i.e., inducing the expression of glucanase in phloem, for example).

Movement of solutes in the phloem is owed to pressure difference between the source tissues and consumer tissues, which are interconnected, because in these solutes, especially sucrose or other sugars and amino acids are rapidly consumed, resulting in water entering the cells by osmosis, and subsequent dilution of the solute; there is also a greater accumulation of solute carbon fixation in the tissue source. The interconnection between both tissues allows the movement of solutes between these tissues. The phloem is also a conduit through which chemical signals are transported between distant organs involved in their communication, wherein said communication is necessary to coordinate the growth and development of vascular plants, both in response to a genetic program and external stimuli.

Transport in the phloem is altered in plants infected with *Candidatus Liberibacter,* particularly, simplasmic transport occurring through cytoplasmic channels or plasmodesmata. *Candidatus Liberibacter* infection causes an excessive accumulation of starch in the leaves; this is reminiscent of mutants in the function of simplasmic transport through the phloem, such as *sxd* in corn (Provencher et al., 1999). *sxd* mutant has a defect in the simplasmic mobilization of sugars, causing starch sugars to polymerize into starch producing the speckle-diffuse asymmetric symptom, such as found in HLB diseased citrus.

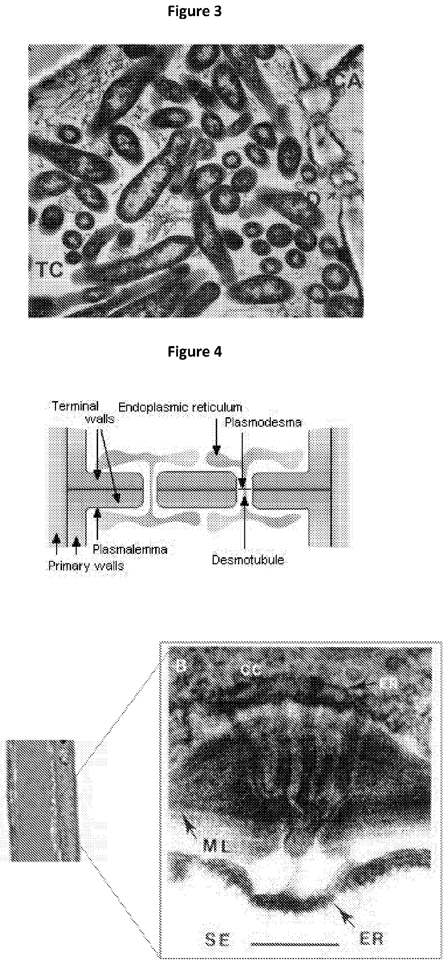

To understand HLB disease it is also important to describe the functions and structures of the conducting tissues of higher plants whose specialization has conferred on them evolutionary advantages, wherein this tissue is formed from the xylem (unidirectional conductor of water and minerals) and the phloem, which translocates photo assimilates, minerals, proteins, and nucleic acids, and consisting essentially of two cell types, the companion cell (CA) and the sieve element (EC), which can be a sieve tube (TC), both of which are interconnected by modified plasmodesmata. The vascular tissue in angiosperms differs from cambium cells, which undergo a longitudinal asymmetric division (Esau, 1953) to lose, when maturating, the nucleus and producing the sieve tube. Figure 2 outlines the vascular tissue of superior plants and the ontogeny of a sieve tube, and its relation to the companion cell. Figure 3 shows the presence of the bacteria in sieve tubes (TC), wherein they establish and settle, while figure 4 shows the structure of plasmodesma (PD), which is the simplasmic connection between the sieve tube and the companion cell, wherein the micrograph shows its appearance when it is obstructed by fibrillar and amorphous material owed to infection. The biochemical identification of these deposits of fibrillar material suggests that they are 1-3 beta glucan (callose) and a "healing" protein called PP2, wherein both molecules are synthesized and deposited on the plasmodesmata as a hypersensitivity reaction.

Plasmodesmata are channels that traverse the membrane and the cellular wall; they are not passive, but specialized and act as dampers to facilitate and regulate communication and transportation of water, nutrients, metabolites, and macromolecules between the plant cells; they contain a form of the endoplasmic reticulum captured, interconnecting all plant cells, except for the guard cells. Plasmodesmata appear to allow the movement of molecules up to 30 kD from both cell types; in contrast, the plasmodesmata interconnecting mesophyll cells have an exclusion limit of about 1 kD.

The mechanisms for transport of macromolecules through the plasmodesma are complex. The model described by Haywood et.al., 2004, considers the presence of structural and facilitating molecules of translocation, wherein a protein recognizing the systemic nature of the molecules forms a complex, which is recognized by a protein anchored to the beginning of the plasmodesma; there should be a wide variety of complexes to recognize families that are so diverse in their sequences, and capable of traversing that simplasmic barrier; chaperon-type molecules unfold proteins and initiate their translocation to the sieve tube. System control is exercised by the nucleus, which transcribes coding RNAs for proteins of supra cellular nature; molecules are folded after traversing the plasmodesma and are in turn recognized by other molecules that presumably will indicate them the target tissue. A great number of proteins have been discovered, which can scroll through plasmodesmata, increasing their exclusion limit, wherein some of these proteins have the ability to bind RNA nonspecifically, while in other cases, their role in the long-distance communication in the phenomenon of floral induction has been demonstrated; there are also background of the ability of RNA to traverse a heterograft and reach distant tissues such as vegetative and floral meristems as reported for squash by Ruiz Medrano and colleagues (1999).

Owed to the spatial restrictions of plasmodesma, there is a molecular exclusion limit as mentioned above, however the presence of proteins of higher molecular weight from 1 up to 250 kD and RNAs suggests that transport is highly selective and regulated by mechanisms not clearly known yet in the entrance of plasmodesma. The nature of the chemical signals involved is largely unknown, but various molecules have been found in the phloem, which could provide such signals; among them are phytohormones and other molecules of low molecular weight, lipids, proteins, and a high diversity of RNA species.

At present experimental approaches are being developed for treating HLB, including the application of pesticides to control the vector. However, their toxicity and residual activity limit their application, while the use of antibiotics attempting to achieve a systematical distribution within the phloem has the limitation that its application must necessarily be continuous, which implies a considerable investment for this strategy.

To control HLB the use of tetracycline has been reported, which interferes with protein synthesis in bacteria, or of penicillin, which inhibits the synthesis of peptidoglicane, a structural component of the bacterial cell wall. Although the result was a marked reduction in symptoms, the use of bacteriostatic tetracycline was discarded because of the phytotoxicity produced thereby; besides, the use of antibiotics in plants and animals for human consumption is not recommended.

Recently, the genome sequence of *Candidatus Liberibacter asiaticus* and also of *Candidatus Liberibacter* ZC, a closely related bacterium which causes the disease known as *Zebra chip* in potatoes, has been obtained; both have high similarity to each other (Hartung et al., 2011; Lin et al., 2011). However, comparison of the sequences available of both pathogens suggests divergent rearrangements in their genomes; wherein the gain and loss of genetic material have contributed to genome differences. Anyhow, available information clearly indicates that *Candidatus Liberibacter* formed a compact clade with a "lifestyle" adapted to the phloem of plants, including e.g., a limited ability to synthesize certain amino acids and nucleic acids. Comparison with the genome of a rhizobacteria, *Sinorhizobium melitoti* shows that although both bacteria are clearly related *Candidatus Liberibacter* has a smaller genome with a much higher A/T content and a limited potential ability to repair DNA deduced from the absence of exonuclease domains on its DNA polymerase and the presence of a single gen for DNA ligase to 10 genes of *S. meliloti* (Hartung et al., 2011).

Studies indicate the susceptibility of *C. Liberibacter* ZC to beta-lactam antibiotics, showing that its cell wall is similar to that of other bacteria having peptidoglycan and therefore susceptible to lysozyme enzyme. Moreover, *C. Liberibacter asiaticus* clearly shows a typical cell wall of Gram-negative bacteria, which also shows that the treatment with beta-lactam antibiotics is possible, a strategy that is being carried out experimentally in the University of Florida and the USDA to control this bacterium (Bové, 2006).

Another strategy that has been considered is to induce the expression of genes involved in systemic acquired resistance or pathogen response. However, from a commercial point of view this is not desirable since the plants will generally reduce productivity in stress situations. Parallel strategies have considered the silencing of key genes in the insect vector. RNA interference (RNAi) to control psyllids would be expressed in the plant by introducing it to the vector when feeding the plant, quenching essential genes in the insect and thereby causing a population decline.

Various strategies have been proposed for the control of bacteria that live in the simplasmic domain and specifically, of the causal agent of HLB. Several laboratories, mainly in the United States, have proposed different strategies to control the bacteria *Candidatus Liberibacter.* For example, the genetic transformation of citrus has been successfully performed via *Agrobacterium tumefaciens*-mediated techniques and biolistics. Table 2 describes the expression of different genes of agronomic interest, for example in citrus species.

**Table 2. Expression of different genes of agronomic interest in citrus species***

| **Common name** | **Scientific name** | **Introduced gene** | **Reference** |
|---|---|---|---|
| Carrizo citrange | *Citrus sinensis x Poncirus trifoliata* | Genes associated with citrus blight | Kayim et al, 2004 |
| | | HAL2 | Cervera, et al, 2000 |
| | | LEAFY APETALA1 | Peña et al, 2001 |
| Grapefruit | *C. paradisi* | Carotenoid biosynthesis genes | Costa et al, 2002 |
| | | VTC capsid protein | Moore, et al, 2000 |
| | | VTC Genes | Febres, et al, 2003 |
| Mexican lime | *C. aurantifolia* | VTC capsid protein | Dominguez et al., 2000 |
| Pokan mandarine | *C. reticulate* Blanco | Ribonuclease chimeric gene | Li et al., 2002 |
| Rangpur lime | *C. limonia* | bO gene (bacteriopsin) | Azevedo et al., 2006 |
| Sour orange | *C. aurantium* | VTC capsid protein | Gutierrez-E et al., 1997; Ghorbel et al., 2000 |
| Trifoliate orange | *P. trifoliata* | Capsid polyprotein pCP | Iwanami et al., 2004 |
| | | Citrus FT (CiFT) Human epidermal growth factor | Endo et al., 2005 Kobayashi et al., 1996 |
| | | rolC | Kaneyoshi and Kobayashi, 1999 |
| Troyer citrange | *C. sinensis x P. trifoliata* | Truncated version of VTC and Bar gene | Piestun et al., 2000 |
| | | rolABC genes | Gentile et al., 2002, Cirvilleri et al., 2005 |
| Valencian orange | *C. sinensis* | Pectin methylesterase gene | Guoert al., 2005 |
| West Indian lime | *Citrus aurantifolia* | Genes to reduce seeds | Koltunow et al., 2000 |

To date, the use of antimicrobial peptides, the induction of the innate immune response, and the use of RNA interference against psyllids have been used with limited results.

Examples of antimicrobial peptides are the myeloid antimicrobial peptide, Beta-defensin-1, Polifemusine-1, Tachyplesin, protegrin-1, Magainin, indolicidin (of animal origin); Cecropins, Sarcotoxin IA, Pirrocoricine (insect originated), and Defensins of plant and animal origin. The expression of these molecules has been evaluated, but the results have not produced the desired control effects.

At present, there are numerous protocols for the genetic transformation of plants with disease control purposes.

For example, patent US6455759 refers to the production of multiple proteins in a transgenic plant, including the DNA construct that expresses two fusion proteins binded by a linker and that are cleaved by enzymes in the plant, both remaining free proteins.

The patent US7196057 refers to the production of fusion proteins for protecting plants from pathogen insects, wherein one part of these proteins is toxic to the insect and the other part thereof translocates toxin through the insect gut, mentioning only the possibility of obtaining transgenic plants expressing the vectors of these fusion proteins without showing evidence of their performance.

Patent application WO2009/064255 relates to the use of a signal anchor that localizes a fusion protein to the apoplast of vascular elements in plant, which may be useful for engineering secretory proteins to the cell wall and/or apoplast of plant cells and for producing secretory proteins in transgenic plant cells as bioreactors.

Patent application WO99/28484 relates to a method for improving resistance or tolerance in a plant and its descendants to a pathogen, consisting of expressing a fusion protein comprising a first domain with anti-pathogenic activity, a linker, and a second domain with anti-pathogenic activity; it also describes the expression constructs, the expression in *E. coli,* its introduction in *Agrobacterium sp* to mediate the transformation of plants and their performance against pathogenic nematodes.

Notwithstanding the above, it is essential to have methods and/or efficient control strategies to address plant diseases caused by microorganisms that invade the phloem via the simplasma, as before the present invention there were no effective control and/or combat procedures.

### Brief description of the invention.

The present invention discloses efficient methods for the treatment of diseases caused by pathogenic microorganisms invading the phloem of plants. Specifically, the invention discloses the genetic modification of citrus explants transformed with *Agrobacterium tumefaciens* containing the citrus CsPP16 gene having SEQ ID No 3, which is homologous to the CmPP16 gene tested in pumpkin (Xoconostle-Cazares et al., 1999) as defined in the claims. This CsPP16 gene was translationally fused via a flexible linker has the sequence of SEQ. ID. No. 4 to antimicrobial peptides selected from the group comprising human alpha-defensin, human lysozyme, indolisine, magainin, cecropin, sarcotoxin, lysozyme, and mixtures thereof wherein the gene that codifies proteins with antimicrobial activity, has a sequence selected from the group comprising SEQ. ID. No. 5, SEQ. ID. No. 8, SEQ. ID. No. 9, SEQ. ID. No. 10, SEQ. ID. No. 11, SEQ. ID. No. 12. For this purpose, a hinge was used that allows CsPP16 proteins and antimicrobial peptides to bend independently. According to the present invention, the proteins used in the control of pathogenic bacteria in the phloem are fused to the amino terminus with the hinge and the supracellular movement protein CsPP16.

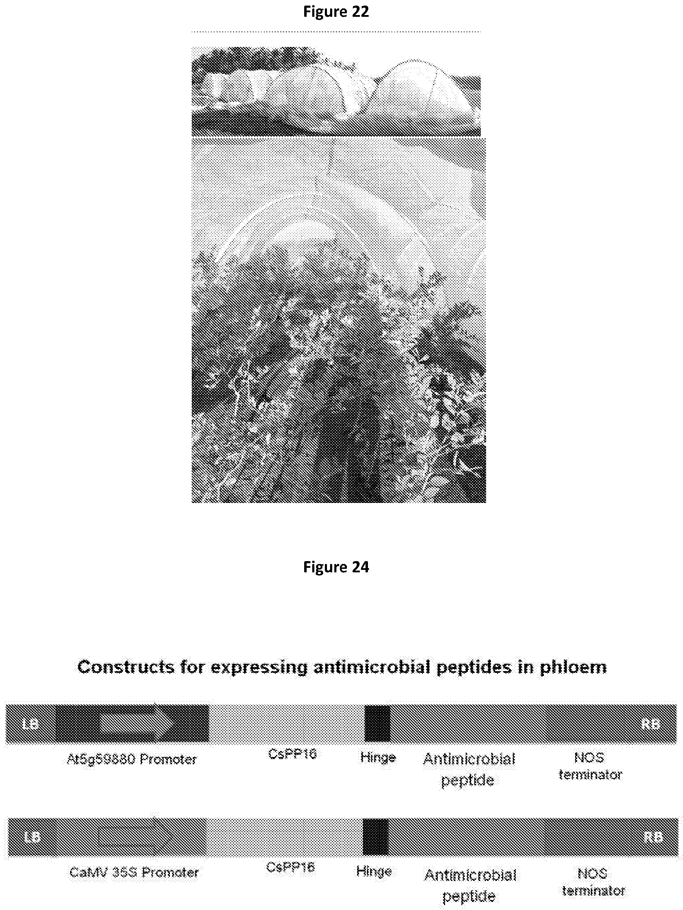

All the genetic constructs as described herein contain the 35S promoter of cauliflower mosaic virus CaMV 35S as promoter 1 and NOS sequence as the terminator sequence. Additionally, another set of constructs containing the phloem specific expression promoter 59880 of *Arabidopsis thaliana* (Ruiz-Medrano et al., 2011) driving the expression of the antimicrobial peptides mentioned above, thereby allowing the expression only in the vascular tissue, where the phytopathogenic bacteria is present. All constructs described herein contain the left and right borders of the T-DNA of *Agrobacterium* on each end, and lack selectable markers (figure 24). The first method of transformation with the constructs described above comprises, for example, the genetic transformation of citrus stems and shoots, which are regenerated *in vitro* to obtain a genetically modified seedling, which is grafted into a rootstock or pattern; for example, of sour orange of *Citrus macrophylla* or *Citrus volkameriana* varieties. The pattern may come from a seedling of one centimeter, for example from the germination of a seed of sour orange, or a pattern of a young citrus plant, which is laterally grafted into a bud whose growth has been genetically modified.

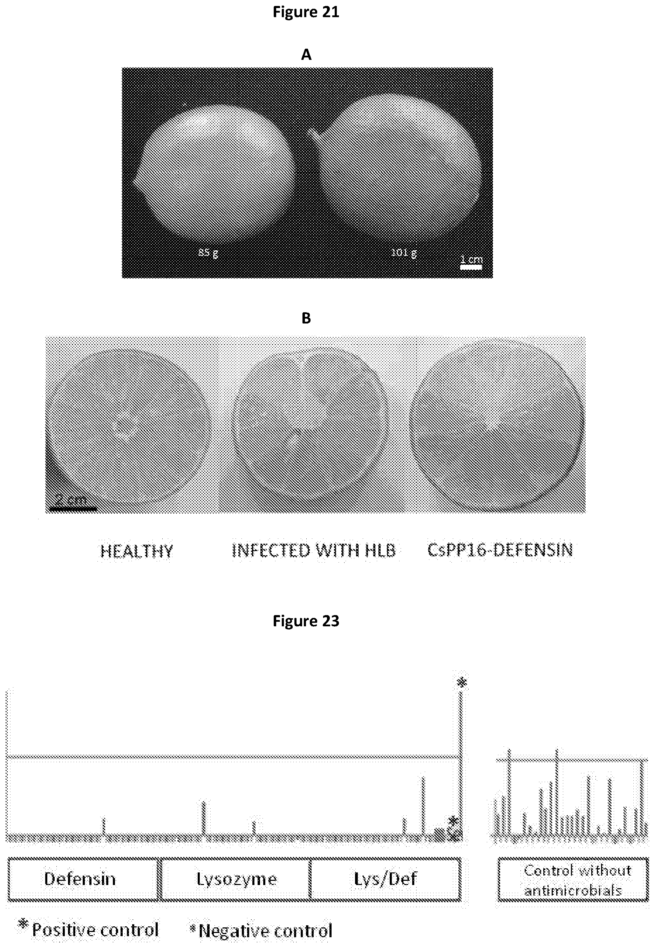

The second method of transformation with the constructs described above comprises promoting the generation of small genetically transformed areas of adult plants, whether healthy or diseased with the bacteria causing HLB. This consists exposing the vascular tissue of stems or branches located between consumer and producer tissues (photosynthetic). This is done by scrapping until observed green photosynthetic tissue, wherein a swab moistened with *Agrobacterium* solution pretreated with acetosyringone and diluted in buffer with plant-growth inducers (auxins and cytokinins) is placed. The treated plant or branch is placed in a bag to maintain the high humidity of the *Agrobacterium tumefaciens* parch up to three days, removing the dressing after the indicated treatment. Plants are exposed to two-day treatments up to a period of one month, obtaining in all cases the expression of the antimicrobials in the vascular tissues and accumulated in the consumer tissues, which are the sites of bacterial infection. According to the present invention, after the transformation of diseased plants, the next step is to monitor the presence of the bacteria causing HLB, which decreases owed to the treatments of expression of the antimicrobials applied alone or in combinations. The diseased plants that were treated by the invention recovered the photosynthesis, which indicates that the vascular tissues were no longer blocked, and the plants were able to flower and fill with fruit. Meanwhile, the plants treated with Defensin flowered as seen in figure 21(A) displaying two limes of 101 and 84 g, which were sliced in their transverse axing, showing symmetrical mesocarp. This is the normal appearance of healthy fruits, compared to those infected, which show asymmetrical sections or mesocarp (figure 21 (B)).

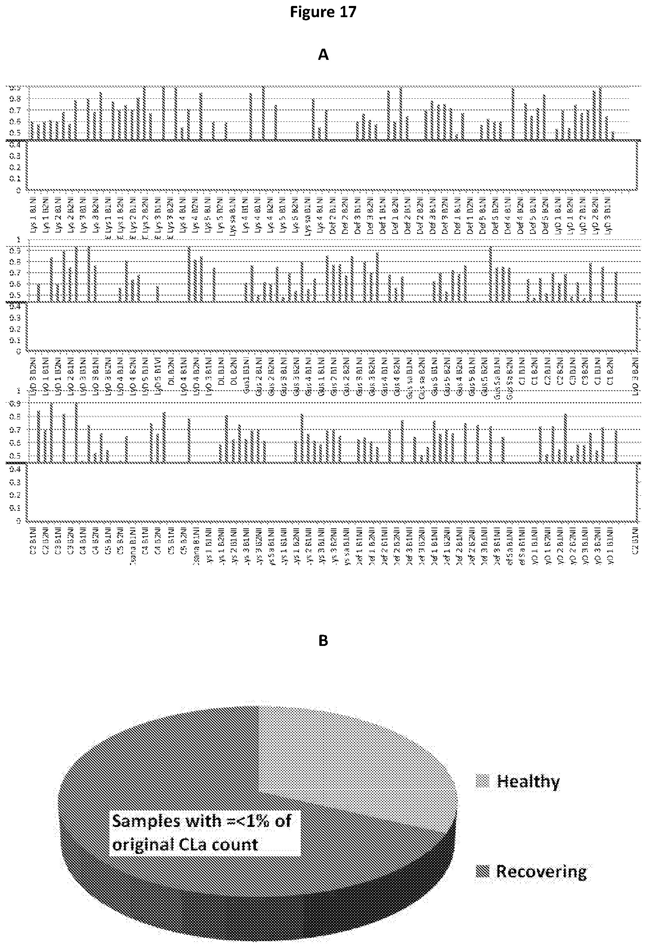

The results obtained in the transformation of 1.20 m diseased plants by the present invention and their evaluation up to 210 days show a substantial reduction of the symptoms of HLB and the recovery of normal physiological conditions of the plant (growth, production of shoots, increasing photosynthesis, flowering, and fruit filling) (figure17).

Moreover, genetic transformation of 1.20 m young trees with a sanitation control certificate, coming from the certified vivarium Emiliano Zapata and exposed to psyllids infected in field with *Candidatus Liberibacter* in Tecoman Colima, Mexico, under biosafety conditions (figure 22), obtained nil or a low amount of bacteria in contrast to the control trees transformed with *Agrobacterium* with plasmids without genes encoding for the antimicrobials mentioned above. The trees described were exposed to psyllids infected with *Candidatus Liberibacter,* and the presence of the bacteria was evaluated after 60 days. This evaluation consisted of collecting two mature photosynthetic leaves and two young leaves, called buds. Total DNA of the central vein was isolated, and the number of bacteria was estimated by real-time PCR of the gene 16S *Candidatus Liberibacter* using fluorescent probes. The obtained values were normalized with the COX endogenous gene of lime (figure 23). The results obtained show that in the treatments with lysozyme, defensin, and a combination of both (Lys and Def), the number of bacteria is reduced or not detectable, in contrast to control plants not expressing antimicrobials.

Moreover, the second method that comprises the transformation of explants and their regeneration via tissue culture according to the present invention shows that the plants are able to regenerate and are vigorous. In these experiments, vigorous grafted plants were obtained by expressing the above antimicrobials, of which ten independent transformants were selected for further field-testing under bio safety conditions.

### Brief description of the figures.

**Figure 1****.** Shows the characteristic symptoms of HLB in trees (upper left) and leaves (upper right), and HLB symptoms in leaves and fruits (bottom). One can observe deformed fruits with aborted seeds and brown vascular bundles (From Strategic Planning for the Florida Citrus Industry, Addressing Citrus, 2010).
**Figure 2****.** Shows the vascular tissue of higher plants, depicting (left) the cross section of a vascular bundle; (A) the ontogeny of a sieve element; (B) the longitudinal cambial cell division; and (C) the asymmetrical growth; (D) the nucleus fragmentation and (E) its disappearance; (F) the accumulation of mucilaginous bodies and (G) the perforation of the cribriform plate in a functional tube. (G) The mature sieve element (right) shows open pores in the cribriform plate, bordered by callose, and P protein dispersed in the peripheral cytoplasm with ER and plastids.
**Figure 3****.** Shows an electron micrograph despicting the presence of *Candidatus liberibacter sp* restricted to the vascular phloem of a citrus tree (From Strategic Planning for the Florida Citrus Industry, Addressing Citrus, 2010). The companion cell (CA), plasmodesma (PD), and sieve tube (TC) are observed.
**Figure 4****.** Shows the location and structure of a plasmodesma. (Upper) The wall site where each pore or desmotubulum traversed by a plasmodesma, where the wall frequently is thicker; traversed tanks of the endoplasmic reticulum close to the wall and associated with the plasmodesma in both cells. (Lower left) The representation of a sieve tube (purple) and a companion cell (pink) can also be observed, and (lower right) a transmission micrograph of a branched plasmodesma present between the companion cell (CA) and the tube or sieve element (EC). Note the endoplasmic reticulum (ER) traversing the plasmodesma.
**Figure 5****.** Shows the evidence of the movement ability of CmPP16 protein (Xoconostle-Cazares et al., 1999). Note that (a) the protein was microinjected into mesophyll cells of *Nicotiana benthamiana* leaves and was able to move from cell to cell; (d and g) when the protein was complexed with RNA, it mediated the RNA movement to neighboring cells. The RNA sense of CmPP16 and the RNA2 of RCNMV are shown as controls, which do not translocate outside the cell themselves. The movement of the labeled protein with another fluorescent molecule (TRITC) to other cells is shown in (e) and (h).
**Figure 6****.** Shows that CmPP16 is synthesized in the companion cell and travels to the sieve tube to coincide with the location of *Candidatus Liberibacter.* (Left) Shows an image of confocal microscopy of an *in situ* hybridization, detecting the RNA encoding the protein CmPP16 located in the vascular bundle (green); and (right) the immunolocalization of protein in purple. Polyclonal antibodies were used and directed against the protein CmPP16 and were revealed with chromogenic alkaline phosphatase, which produces the purple color (Xoconostle-Cazares et al., 1999).

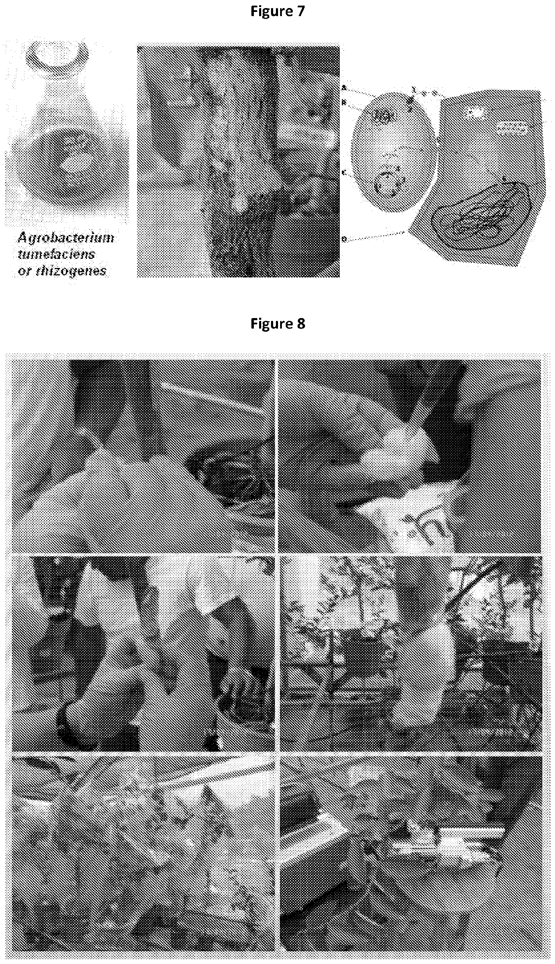

**Figure 7****.** Shows the transformation of mature citrus plants, particularly the transformation of stems with *A. tumefaciens.* Initially (left), the *in vitro* culture is aerobically grown in the bacteria containing the gene encoding the antimicrobial proteins. Separately (center), the bark of the trees to be transformed is removed to expose the vascular tissue. The diagram (right) shows that (1) the cell recognizes the plant signals, which are compounds of low molecular weight; (2) these compounds are recognized by the bacteria via a two-component Phospho-relay system; (3) the synthesis of T-DNA present in the binary plasmid is induced, resulting in a single strand of T-DNA, which forms proteins complexes; (5) additional virulence genes are switched on to help mobilize (6) the T-DNA into the plant cell. In this process, a copy of the T-DNA is stably inserted into the genome of the plant cells.
**Figure 8****.** Shows the procedure of transformation of the present invention by *A.* tumefaciens and A. rhizogenes of mature cells from mature plants of Mexican lime. In the greenhouse (upper left) the stem bark to be infected is removed (upper right), and the swab is moistened with the solution containing *A. tumefaciens* or *A. rhizogenes.* Subsequently (center left), the swab is placed on the stem, which (center right) is wrapped in flexible plastic and secured with tape. The plants (lower left) are transferred to plastic bags to maintain high relative humidity, which favors their genetic transformation. After the transformation (lower right), the photosynthetic capacity of the plants is evaluated as an indication that their vascular bundles are permeable.
**Figure 9****.** Shows the development of small protruding photosynthetic areas in form of calluses in the plants inoculated according to the present invention (left). In the right panel, tumors were delineated to visually locate them.

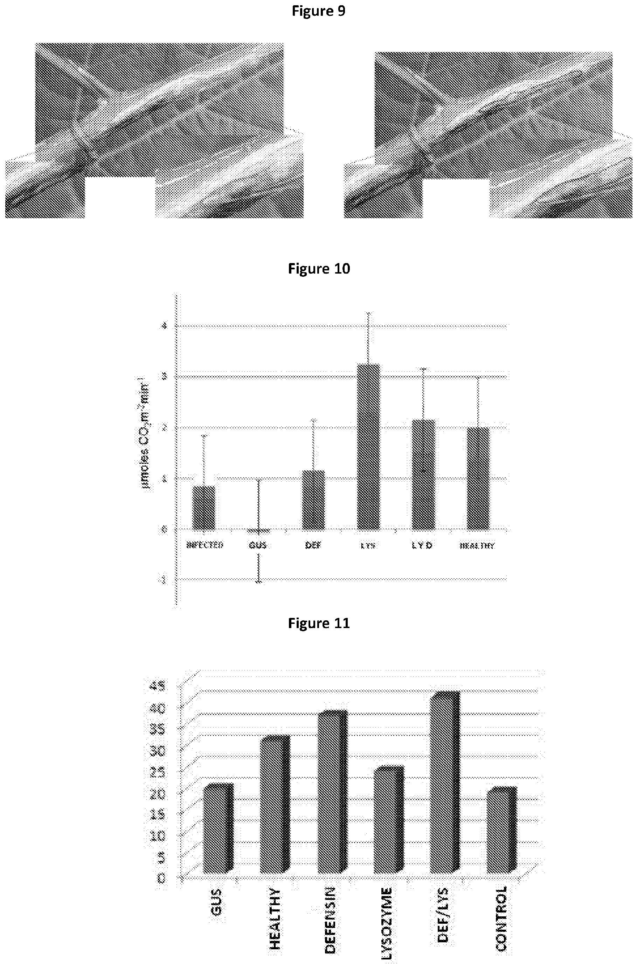

**Figure 10****.** Shows the estimating photosynthesis of plants treated according to the present invention. The photosynthesis was measured to 500 micromoles light intensity. The photosyntesis values correspond to fixed micromoles of CO₂ per cm⁻¹ x seg⁻¹. The control corresponds to the average values of five infected plants inoculated with water. The GUS bar corresponds to transformed plant infected with the GUS gene (which encodes beta-glucuronidase). The DEF bar corresponds to the transformation with the gene encoding the defensin fused with CsPP16, LIS bar, lysozyme-CsPP16, and LYD-CsPP16, the combination of both antibacterials. Healthy lime plants were used as control. As shown, after the treatment the lime plants infected and transformed with antimicrobials increase their photosynthetic capacity.
**Figure 11****.** Shows the number of new shoots in plants treated according to the present invention. The graph indicates the size of new shoots in treated plants, wherein the plants with larger shoots were those receiving the treatments with antimicrobials according to the invention. The treatments were: GUS (plants expressing a reporter protein without antimicrobial capacity); DEF/LIS (plants expressing human defensin fused with CsPP16 and lysozyme fused with CsPP16; both genes are expressed from the CaMV 35S promoter and are flanked by the left and right borders of T-DNA).

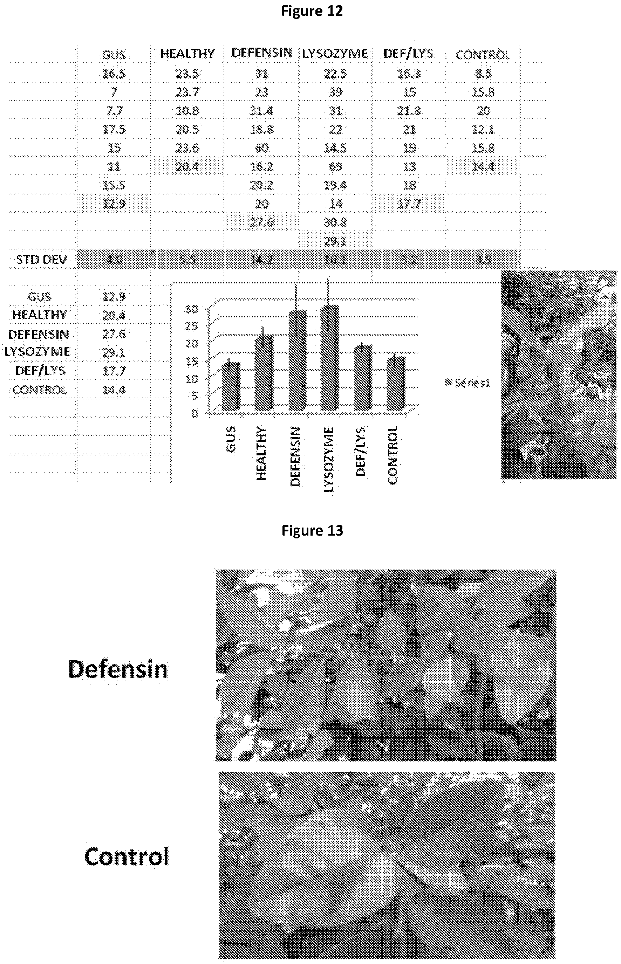

**Figure 12****.** The upper table shows the size of the new shoots in the plants after the indicated treatment. The bottom chart shows the average length of the new shoots. The plants with larger shoots were those receiving the treatments with antimicrobials according to the present invention. (Bottom left) Shows the appearance of new shoots without symptoms in plants treated with antimicrobials. The treatments were: GUS (plants expressing a reporter protein without antimicrobial capacity); DEF/LIS (plants expressing human defensin fused with CsPP16 and Lysozyme fused with CsPP16; both genes are expressed from the CaMV 35S promoter and are flanked by the left and right borders of T-DNA).
**Figure 13****.** Shows the appearance of the treated plants after 100 days of treatment.
**Figure 14****.** Shows the comparison of the leaf areas of plants before and after the treatment with antimicrobials according to the present invention.
**Figure 15****.** Shows the development of small tumors hyperproducers of antimicrobials in the stems of the plants produced by the genetic transformation according to the present invention.
**Figure 16****.** Shows the quantification of *Candidatus Liberibacter* bacteria at 60 days in plants treated with antimicrobials according to the present invention. The data are arbitrary units calculated by real-time PCR.
**Figure 17****.** Shows (A) the detection of *Candidatus Liberibacter* in adult plants infected with HLB by quantitative PCR. The bacterial load was measured by real-time PCR; the data were normalized by COX citrus endogenous gene. The highest value was assigned the arbitrary value of 1 (untreated plants). The yellow asterisk shows the value obtained in healthy plants. Red asterisks show the plants that being initially diseased decreased the bacterial load comparable to healthy plants. (B) Simplified diagram of the evaluation at 210 days of treatment, wherein more than a third of the plants are healthy, while the remaining two-thirds decreased 99% the bacterial content.
**Figure 18****.** Shows the *in vitro* culture of Mexican lime seeds. We can observe (left) day 1, (center) 3^{rd} week incubated at room temperature in the dark and (right) 5^{th} week incubated at room temperature in the presence of light.
**Figure 19****.** Shows the regeneration process of explants transformed with *A. rhizogenes* and *A. tumefaciens* according to the present invention. We can observe (upper left) explants on co-culture medium, (upper right) explants on regeneration medium (SRM) for 30 days in darkness conditions and (bottom) explants in regeneration medium (SRM) for 60 days in light-darkness conditions.
**Figure 20****.** Shows the development and regeneration of a graft (left) at 15 days (center) and 60 days (right) after the generation of grafts in different patterns.
**Figure 21****.** Shows limes obtained from HLB diseased plants and subjected to treatment with defensin antimicrobial according to the present invention. We can observe (A) the appearance of limes expressing defensin (note that their weight, 101 and 84 g, is compared to the weight of a commercial lime of an average weight of 90 g); (B) a healthy lime (left); a lime infected with irregular segments and smaller fruits (center) and a lime expressing CsPP16-defensin (right), wherein the symmetry of the mesocarp is comparable to that of healthy fruits.
**Figure 22****.** Shows the appearance of the assay of 120 healthy plants transformed with CsPP16-lysozyme, CsPP16-defensin, and a combination thereof. It was performed in microtunnels covered with anti-aphid mesh in Tecoman, Colima, Mexico, HLB endemic area (top). The two left panels contain 160 plants treated with the antimicrobial and exposed to psyllid infected with *Candidatus Liberibacter* bacteria, CsPP16-defensin, CsPP16-indolisin, CsPP16-magainin, CsPP16-cecropin, CsPP16-sarcotoxin, CsPP16-lysozyme, and pair combinations thereof. Shows (bottom) the interior of a tunnel.
**Figure 23****.** Shows the quantification of the bacteria *Candidatus Liberibacter* in young Mexican lime trees expressing CsPP16-defensin, CsPP16-lysozyme, and both at 60 days after exposure to infected psyllids. Antimicrobial treatments according to the present invention (left) show a lower amount of bacteria and were compared to the negative control. In contrast, the control plants without antimicrobials (right) show generally a higher amount of bacteria. In defensin, we detected at low levels the bacteria in only one plant, whereas in the treatment with lysozyme, we detected two plants, also at low levels. In the combination, we can see the detection of *Candidatus Liberibacter* in two plants. These plants correlate to low antimicrobial levels.
**Figure 24****.** Shows the protein expression units with antimicrobial activity according to the present invention, capable of expressing said proteins in phloem. We can see (top) the expression unit containing the AT5G59880 promoter and (bottom) the expression unit containing the CaMV 35S promoter.
**Figure 25****.** Shows the decrease of CLa bacteria associated to HLB disease, which was detected by real-time EMA-PCR.
**Figure 26****.** Shows the acute toxicological analysis in BALB/c mice with lime-juice from fruits of the plants treated according to the present invention.
**Figure 27****.** Shows the appearance of adult Mexican lime trees under confinement at 3 months of antimicrobial treatment according to the present invention.
**Figure 28****.** Shows the appearance of the plants grown in microtunnels after 12 months (Mexican lime) and 6 months of treatment (Persian lime, orange, grapefruit, and mandarine).
**Figure 29****.** Shows the appearance of the plants grown under biocontainment.
**Figure 30****.** Shows the lime plants treated according to the present invention.
**Figure 31****.** Shows the bacteria content (left) at 3 months of treatment in shoots and mature leaves of infected plants treated according to the present invention and the initial and final photosynthesis (right) of plants treated with defensin (treatment 1), lysozyme (treatment 2), and a mixture of defensin and lysozyme (treatment 3).
**Figure 32****.** Shows the leaf area of (A) orange, (B) grapefruit, (C) Persian lime, and (D) mandarine plants treated according to the present invention. We can see the leaf area in mature (left bar of each group) and young plants (right bar of each group).
**Figure 33****.** Shows the bromatological parameters obtained for lime-juice from fruits of the trees treated with defensin, lysozyme and a mixture thereof, in addition to limes from control trees.
**Figure 34****.** Shows the defensin immunodetection in the vascular tissue of a genetically modified lime. We can observe the (X) xylem, (C) cambium, and (F) phloem (companion cells and sieve tubes).
**Figure 35****.** Shows an end-point PCR assay of the detection of genes encoding antimicrobials in the plants treated with *Agrobacterium tumefaciens* transformed with the above mentioned constructs. We can observe a fragment of 402 bp, which corresponds to the gene encoding for human lysozyme.
**Figure 36****.** Shows (A) the appearance of pollen from plants treated with defensin (RR09) and control plants untreated; (B) the average area of pollen untreated (left bar) and with antimicrobial treatment (lysozyme) (right bar).

### Detailed description of the invention.

The present invention relates to efficient methods for the treatment of diseases caused by pathogenic microorganisms invading the phloem of plants, particularly to methods for the generation of transgenic plants, including citrus, resistant to bacterial infection by the clade of *Candidatus Liberobacter (Ca.L.)* that cause HLB disease; these bacteria are located in a restricted manner in the phloem; therefore, the protocols where antibiotics are used have been unsuccessful.

Until the present invention, the solution to this disease had not been addressed by genetic engineering methods whereby the generated plants will acquire a genetic construct, which is stably found and expressed.

It is considered that the most effective method for long-term management of diseases associated with Liberibacteria relies on the acquisition of resistance in the host plants, whereby the present invention is based on the acquisition of resistance in plants using genetic engineering. Therefore, the invention comprises a method of genetic transformation, which is adequate for citrus species and is directed to transfer a defense mechanism to eliminate or combat the infection caused by at least one pathogenic microorganism invading the phloem of the plants; for example, bacteria of the group comprising *Candidatus Liberobacter, Candidatus Liberobacter asiaticus, Candidatus Liberobacter africanus,* and/or *Candidatus Liberobacter americanus,* thereby controlling the diseases associated with these infections, such as HLB and Zebra chip (ZC). The success of the method of the present invention is based on using a mechanism used by plants to translocate molecules with antimicrobial activity via the vascular tissue.

The present invention is based, e.g., on the fact that the cell wall of *Candidatus Liberibacter spp.* is composed of peptidoglycan, so we assume that the bacteria are susceptible to be destroyed by enzymes such as lysozyme. Also, the present invention relates to a strategy for controlling the infection by using the need of *Candidatus liberibacter spp.* to settle in the phloem of the plant.

The method of the present invention comprises to achieving the expression of a chimeric gene encoding a fusion protein that has a double function: to act as a transporter inside the vascular tissue of the plant and to perform antimicrobial activity; e.g., to destroy the cell wall of pathogenic bacteria invading the phloem of the plants, for example *Candidatus Liberibacter spp.* During the development of the present invention, we conducted a transformation protocol with which it was possible to obtain a reasonable transformation efficiency and a successful and stable integration of the chimeric transgene proposed herein, as confirmed by the experimental data shown below, which also show that the protection against HLB infection by the method of the present invention has also been successfully owed to the transference, the expression, and the functionality of the transferred gene.

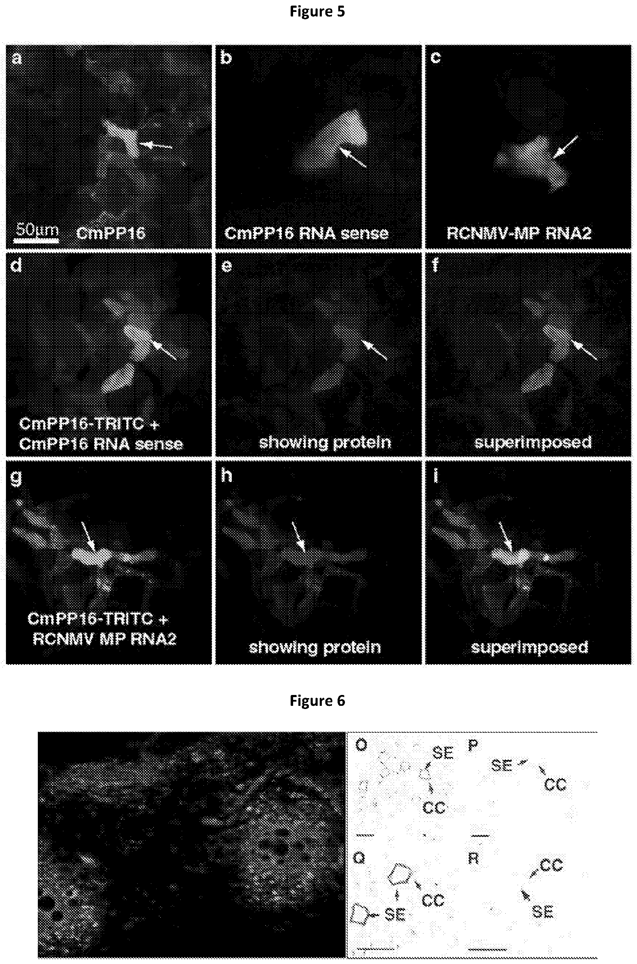

Our research team has characterized in detail a protein that binds RNA independent of its sequence; we have called it CmPP16, wherein this protein not only associates to different RNAs *in vitro,* but is also capable to transport them from one cell to another via the plasmodesmata (Xoconostle-Cazares et al., 1999). CmPP16 is therefore, functionally similar to the movement proteins of RNA viruses although, as it has already been implied, its role in healthy plants is unknown. This pioneering work demonstrated the presence of an endogenous system of the healthy plant to translocate proteins and nucleic acids, a system that is used by viruses to move systemically. Homologues of the protein CmPP16 have been found in different plant species, but their conservation is not as high. Our investigations also indicate that CmPP16 fused to larger proteins does not lose its ability to intercellular transport via plasmodesmata. An evidence of the movement ability of the protein CmPP16 are the results shown in figure 5, wherein the images of confocal microscopy show that when CmPP16 protein is microinjected into mesophilic cells of *Nicotiana benthamiana* leaves can move from cell to cell; and when CmPP16 was complexed with RNA, it also mediated the movement of RNA to neighboring cells. The figure 6 shows the location of CmPP16 in the phloem.

Regarding pathogens circulating through the phloem and rarely leave it during the infectious process, a limitation on the treatment of diseases caused by these pathogens is the inability of chemical agents (e.g., antibiotics) to reach the sieve elements harboring the pathogen. Based on this precedent, for the development of the present invention we have considered that the protein CsPP16 having SEQ ID No 3, functions as vehicle to transport proteins into the sieve elements, which are normally not accessible. Therefore, we suggest that a protein with antimicrobial activity as defined in the claims, fused to CsPP16 as defined in the claims, could access the phloem and particularly the sieve element, wherein the peptide exerts its antimicrobial and/or antibacterial activity. For succeeding in the strategic approach of the present invention, it was necessary to design the expression vector of the fusion proteins CsPP16-peptide described herein and to perform a transformation strategy.

The present invention proposes the use of a gene encoding the protein CsPP16 fused with a protein with antimicrobial activity, for example with antibacterial activity; in this case, for example human lysozyme as defined in the claims. The expression of such fusion protein is to be directed by a phloem-specific promoter, for example which we have previously characterized. Said promoter regulates the expression of a protein that is required to maintain the high conductivity of the phloem such as for example the actin-depolymerizing factor 3 (ADF3), although other factors achieving the same effect can be used in the present invention.

Based on the literature, we selected proteins with antimicrobial activity in other systems, selected those with antimicrobial activity in plants. It is worth noting that we used human lysozyme and human defensin to prevent the recombinants from generating immune problems in humans, since the citrus fruits are intended for human consumption.

According to the present invention, the antibiotic protein sequences were obtained from the GenBank database (NCBI), and the amino acid sequence was converted to a base sequence using the more common codons in citrus. The information required to change the usage of codons is in table 3.

To provide the essential elements to be able to carry out the invention, the list of commonly used codons for citrus available in the database http://www.kazusa.or.jp/codon/ is presented below.

| | |
|---|---|
| *Indian citrus ringspot virus* [gbvrl]: 12 | *Citrus idaeovirus* [gbvrl]: 1 |
| *Citrus sinensis x Poncirus trifoliata* [gbpln]: 3 | *Citrus tatter leaf virus* [gbvrl]: 3 |
| *Citrus natsudaidai* [gbpln]: 1 | *Cytoplasmic citrus leprosis virus* [gbvrl]: 18 |
| *Citrus leaf blotch virus* [gbvrl]: 3 | *Citrus leaf rugose virus* [gbvrl]: 4 |
| *mitochondrion Citrus junos* [gbpln]: 1 | *Citrus variegation virus* [gbvrl]: 17 |
| *Citrus junos* [gbpln]: 7 | *Citrus maxima* [gbpln]: 8 |
| *Citrus sinensis x Citrus reticulata* [gbpln]: 1 | *Citrus x paradisi* [gbpln]: 28 |
| *Citrus aurantiifolia* [gbpln]: 1 | *Citrus kinokuni* [gbpln]: 1 |
| *Citrus latipes* [gbpln]: 1 | *Citrus cv. Shiranuhi* [gbpln]: 12 |
| *Citrus hystrix* [gbpln]: 1 | *Citrus unshiu* [gbpln]: 64 |
| *Microcitrus sp. citruspark01* [gbpln]: 1 | *Mitochondrion Citrus jambhiri* [gbpln]: 1 |
| *Citrus hybrid cultivar* [gbpln]: 1 | *Citrus jambhiri* [gbpln]: 15 |
| *Citrus yellow mosaic virus* [gbvrl]: 6 | *Citrus psorosis virus* [gbvrl]: 9 |
| *Citrus limon* [gbpln]: 11 | *Citrus cv. Sainumphung* [gbpln]: 5 |
| *Chloroplast Citrus sinensis* [gbpln]: 89 | *Citrus iyo* [gbpln]: 2 |
| *Citrus sinensis* [gbpln]: 116 | *Citrus reticulata* [gbpln]: 3 |
| *Citrus macrophylla* [gbpln]: 2 | *Citrus clementina* [gbpln]: 4 |
| *Citrus sudden death-associated virus* [gbvrl]: 4 | *Citrus ringspot virus* [gbvrl]: 1 |
| *Citrus tristeza virus* [gbvrl]: 476 | *Citrus clementina x Citrus reticulata* [gbpln]: 4 |

For the design of the constructs used in the present invention, after obtaining the sequence of the synthetic genes, these were added with regulatory sequences, for example the promoter 35S of the cauliflower mosaic virus and NOS terminator. However, other regulatory sequences that work in the same way can be used for the purposes of the present invention.

**Table 3. Usage of common codons in Citrus sinensis x Citrus reticulate**

| ***Citrus sinensis* x *Citrus reticulata* [gbpln]: CD's (354 codons)** | | | |
|---|---|---|---|
| **Fields: [triplet] [frequency: by thousands] ([number])** | | | |
| UUU 19.8 (7) | UCU 14.1 (5) | UAU 22.6 (8) | UGU 5.6 (2) |
| UUC 19.8 (7) | UCC 8.5 (3) | UAC 16.9 (6) | UGC 8.5 (3) |
| UUA 2.8 (1) | UCA 14.1 (5) | UAA 0.0 (0) | UGA 0.0 (0) |
| UUG 22.6 (8) | UCG 2.8 (1) | UAG 2.8 (1) | UGG 8.5 (3) |
| CUU 22.6 (8) | CCU 14.1 (5) | CAU 28.2 (10) | CGU 2.8 (1) |
| CUC 16.9 (6) | CCC 14.1 (5) | CAC 2.8 (1) | CGC 0.0 (0) |
| CUA 16.9 (6) | CCA 19.8 (7) | CAA 28.2 (10) | CGA 2.8 (1) |
| CUG 19.8 (7) | CCG 0.0 (0) | CAG 5.6 (2) | CGG 5.6 (2) |
| AUU 42.4 (15) | ACU 8.5 (3) | AAU 25.4 (9) | AGU 16.9 (6) |
| AUC 19.8 (7) | ACC 11.3 (4) | AAC 16.9 (6) | AGC 19.8 (7) |
| AUA 8.5 (3) | ACA 14.1 (5) | AAA 25.4 (6) | AGA 8.5 (3) |
| AUG 42.4 (15) | ACG 2.8 (1) | AAG 42.4 (15) | AGG 5.6 (2) |
| GUU 33.9 (12) | GCU 22.6 (6) | GAU 36.7 (13) | GGU 36.7 (13) |
| GUC 11.3 (4) | GCC 19.8 (8) | GAC 25.4 (9) | GGC 14.1 (5) |
| GUA 11.3 (4) | GCA 14.1 (1) | GAA 28.2 (10) | GGA 22.6 (8) |
| GUG 14.1 (5) | GCG 2.8 (1) | GAG 19.8 (7) | GGG 5.6 (2) |

| | | | |
|---|---|---|---|
| NOTE: When encoding GC 43.22% 1^{st} letter GC 51.98% 2^{nd} letter GC 34.75% 3^{rd} letter GC 42.94% | | | |

All the genetic constructs described herein contain as promoter 1 the d35S promoter of the cauliflower mosaic virus CaMV 355 and the NOS sequence as terminator sequence. Additionally, another set of constructs contains the phloem-specific expression promoter 59880 of *Arabidopsis thaliana* for conducting the expression of the antimicrobial peptides mentioned above, thereby allowing the expression only in vascular tissue where the phytopathogenic organism for example, is found. All constructs described herein contain in their ends the left and right borders of the T-DNA of *Agrobacterium* and lack selection markers.

The main objective of the present invention is to provide methods for obtaining genetically modified or transgenic plants expressing a fusion protein by which they acquire the resistance vs. microorganism's infections affecting the phloem of the plants and generating diseases, such as the bacteria causing HLB. One of the methods of the invention comprises following steps:
**1)** Conjugated to a strain of *Agrobacterium* a DNA expression vector of CsPP16 protein fused to an antibacterial molecule,
**2)** Inoculate a plant susceptible to infection by microbial agents that cause diseases, for example bacterial agents causing HLB or diseases caused by microorganisms affecting the phloem of the plants, with the said strain of *Agrobacterium* that possesses the expression vector.
**3)** Verify the acquisition of the vector and its expression, its stability in the transformed plants and its descendants, and verify the resistance acquired.

In one embodiment, the above method comprises in step 2) the inoculation of the previously infected plants with the antimicrobial agent causing the disease, with the vectors described herein.

According to the present invention, the expression vector construction is provided as defined in the claims, which should possess basically the gene encoding CsPP16 having SEQ ID No 3 linked through a flexible linker has the sequence of SEQ. ID. No. 4 to the gene coding for an antimicrobial protein having a a sequence selected from the group comprising SEQ. ID. No. 5, SEQ. ID. No. 8, SEQ. ID. No. 9, SEQ. ID. No. 10, SEQ. ID. No. 11, SEQ. ID. No. 12. with specificity for removing microorganisms affecting the phloem of the plants, for example bacteria located in the phloem of the plants, particularly bacteria such as *Candidatus Liberibacter, Candidatus Liberibacter asiaticus, Candidatus Liberibacter africanus,* and/or *Candidatus Liberibacter americanus,* which cause HLB infection in citrus species. In the embodiments of the invention also are those expression vectors that facilitate the expression of a fusion protein selected from the group comprising CsPP16-peptide with antimicrobial activity, CsPP16-myeloid antimicrobial peptide, CsPP16-lysozyme, CsPP16-Beta-defensin-1, CsPP16-Polyphemusin-1, CsPP16-Tachyplesin, CsPP16-Protegrin-1, CsPP16-Magainin, CsPP16-Indolicidin, CsPP16-Cecropin, CsPP16-Sarcotoxin IA, CsPP16-Pyrrocorycin and CsPP16-Defensins, and a mixture thereof.

Another embodiment of the invention is the possibility of generating resistant to "Zebra chip" (ZC) condition that occurs in potatoes by *Candidatus Liberibacter solanacearum.*

Still, other embodiments of the invention are the expression vectors constructions shown in figure 24; they are characterized by having the following sequences:
- CsPP16-Lysozyme expression unit, containing the T-DNA left border of *A. tumefaciens* (SEQ. ID. No. 1), the short version of the 35S promoter of the cauliflower mosaic virus (SEQ. ID. No. 2), the gene encoding the 16K protein of the phloem of *Citrus aurantifolia,* orthologous in citrus or CsPP16 protein (SEQ. ID. No. 3), a flexible poly linker (SEQ. ID. No. 4), human lysozyme with codon usage in citrus (SEQ. ID. No. 5) and the NOS terminator and the T-DNA right border of *A. tumefaciens* (SEQ. ID. No. 6).
- CsPP16-Defensin expression unit, containing the T-DNA left border of *A. tumefaciens* (SEQ. ID. No. 1), the large promoter 35S of the cauliflower mosaic virus (SEQ. ID. No. 7), the gene encoding the 16K protein of the phloem of *Citrus aurantifolia,* orthologous in citrus or protein CsPP16 (SEQ. ID. No. 3), a flexible polylinker (SEQ. ID. No. 4), human defensin with usage of codons in citrus (SEQ. ID. No. 8), the NOS terminator and the T-DNA right border of *A. tumefaciens* (SEQ. ID. No. 6).

Also, and for purposes of the present invention, following expression units are also comprised:
- CsPP16-Magainin expression unit containing all the elements indicated for the CsPP16-Defensin expression unit, except human Defensin with codon usage in citrus (SEQ. ID. No. 8), which is replaced by Magainin with codon usage in citrus (SEQ. ID. No. 9).
- CsPP16-Cecropin expression unit containing all the elements indicated for the CsPP16-Defensin expression unit, except human Defensin with codon usage in citrus (SEQ. ID. No. 8), which is replaced by Cecropin with codon usage in citrus (SEQ. ID. No. 10).
- CsPP16-Sarcotoxin expression unit containing all the elements indicated for the CsPP16-Defensin expression unit, except human Defensin with codon usage in citrus (SEQ. ID. No. 8), which is replaced by Sarcotoxin with codon usage in citrus (SEQ. ID. No. 11).
- CsPP16-Indolicidin expression unit containing all the elements indicated for the CsPP16-Defensin expression unit, except human Defensin with codon usage in citrus (SEQ. ID. No. 8), which is replaced by Indolicidin with codon usage in citrus (SEQ. ID. No. 12).

In one of the embodiments of the invention, the short promoter 35S (SEQ. ID. No. 2) or large promoter (SEQ. ID. No. 7) of the cauliflower mosaic virus found in the expression units of the invention CsPP16-Lysozyme, CsPP16-Defensin, CsPP16-Magainin, CsPP16-Cecropin, CsPP16-Sarcotoxin, and CsPP16-lndolicidin mentioned above, can be replaced by the vascular promoter AT5G59880 (SEQ. ID. No. 13).

The expression units mentioned above were later synthesized by GenScript USA Inc., sequenced to verify that no mutations were inserted in the assembly process and cloned into the vector with resistance to beta-lactam antibiotic. The plasmids obtained were transformed by electroporation into the bacteria *Agrobacterium tumefaciens* and *Agrobacterium rhizogenes,* selecting the transforming bacteria with the antibiotic carbenicillin at final concentration of 100 µ/ml.

The first method of transformation with the constructs described above comprises, for example, the genetic transformation of citrus stems and shoots, which are regenerated *in vitro* to obtain a genetically modified seedling, which is grafted into a rootstock or pattern, such as sour orange, and volkameria or macrophylla varieties. The pattern may come from a seedling of 1 cm, from the germination of a seed of sour orange, or the pattern is a young citrus plant, to which the genetically modified bloom is laterally grafted for its growth.

The second method of transformation with the constructs described above comprises to promote the generation of small genetically modified areas of adult plants, healthy or diseased with the bacteria producing HLB. This involves exposing the vascular tissue of stems or branches located between consumer and producer tissues (photosynthetic). This is done by scraping till finding the green photosynthetic tissue and placing therein a swab moistened in buffer with a solution of *Agrobacterium* pretreated with acetosyringone and diluted in buffer with plant-growth inducers (auxins and cytokinins). The treated plant or branch is placed in a bag to maintain in high humidity the parchment with *Agrobacterium tumefaciens* up to three days, removing the dressing after the indicated treatment. Plants are exposed to treatments of two days up to a month, obtaining in all cases the expression of the antimicrobial in the vascular tissues and accumulated in consumer tissues, which are the sites of infection of the bacteria. According to the present invention, after the transformation of diseased plants, the presence of the organism causing the disease is monitored; for example, the bacteria causing HLB, which decrease owed to the treatments of antimicrobial expression applied alone or in combinations. Diseased plants that were treated by the invention recovered their photosynthesis, indicating that the vascular tissues were no longer blocked and the plants were able to blossom and conduct a proper filling of fruit. Meanwhile, the plants treated with Defensin flowered; the figure 21A shows two limes obtained, 101 and 84 g, which were cut in their transverse axis, showing a symmetrical mesocarp. This appearance is normal in healthy fruits, compared to those infected, which contain an asymmetrical mesocarp (segments) (figure 21B).

The results in the transformation of diseased plants of 1.20 m high by the present invention and their evaluation up to 210 days, show a substantial decrease in the symptoms of HLB and the recovery of normal physiological conditions of the plant (growth, production of shoots, increasing photosynthesis, flowering, and fruit filling) (figure 17).

Moreover, genetic transformation of 1.20 m young trees, with sanitation certificate and from the certified vivarium Emiliano Zapata, were exposed to psyllids infected in field with *Candidatus Liberibacter* in Tecoman, Colima, Mexico, under bio safety conditions (figure 22) obtained null or a low amount of bacteria, in contrast to the control trees that were transformed with *Agrobacterium,* with plasmids without genes encoding the antimicrobials mentioned above. Trees described were exposed to psyllids infected with *Candidatus Liberibacter,* and the presence of the bacteria was evaluated after 60 days. This evaluation consisted of collecting 2 mature photosynthetic leaves and 2 young leaves, called buds. Total DNA of the central vein was isolated, and the number of bacteria was estimated by real-time PCR of the gene 16S of *Candidatus Liberibacter* using fluorescent probes. The obtained values were normalized with the endogenous gene of COX lime (figure 23). The results show that in the treatment with lysozyme defensin, and a combination thereof (Lys and Def), the number of bacteria is reduced or not detectable, in contrast to control plants not expressing the antimicrobials.

Moreover, the second method comprising the transformation of explants and their regeneration via tissue culture according to the present invention shows that the plants are able to regenerate and are vigorous. In these experiments, vigorous grafted plants were obtained by expressing the aforementioned antimicrobials, of which ten independent transformants were selected for further field-testing under bio safety conditions.

Below, we describe as examples two constructs used that include the sequences fused, which were synthesized, and examples of the procedure to obtain genetically transformed plants resistant to HLB. We also include a description that uses examples to determining the performance of the constructs as vectors for the transfer of the designed fusion genes.

The following examples are included for the sole purpose of illustrating the invention, without implying limitations on its scope.

**Example 1. Obtaining CsPP16 expression units containing the 35S promoter of the cauliflower mosaic virus.** To obtain such expression units of the sequences indicated in table 4, they were synthesized and assembled in the order listed; they were sequenced to verify that no mutations were inserted in the assembly process and finally cloned into the vector of Genscript, containing carbenicillin antibiotic resistance.

The resulting recombinant plasmids were transformed into competent cells of *Agrobacterium tumefaciens* and *A. rhizogenes.* We used carbenicillin antibiotic, which is the synthetic version of ampicillin and is more stable. Plasmid DNA was extracted from the resistant bacteria, and the presence of the recombinant plasmid was verified on agarose gel.

To verify the presence of the gene of interest with antimicrobial activity inserted into the vector or on the plant thereby transformed, specific oligonucleotide primers were used for PCR amplification; for example, in the case of lysozyme, the forward oligonucleotide 3'-AGGTTTTTCGAAAGATGCGAACTTGCTAGAA-5' (SEQ. ID. No. 14), and the reverse 3'-AAACACCGCAACCTTGAACATATTGTCTGC-5' (SEQ. ID. No. 15); and in the case of defensin, the forward oligonucleotide 3'- ATGAGAGTTCTTTATCTTCTTTTCAGCTTC-5' (SEQ. ID. No. 16) and the reverse 3'-ACTTCTTCTTGCAGCATCTTGTACCTGGAA-5' (SEQ. ID. No. 17).

**Example 2. Obtaining CsPP16 expression units containing the vascular promoter AT5G59880.** For obtaining such expression units, the promoter 35S of the cauliflower mosaic virus, the short version (SEQ. ID. No. 2) or the large version (SEQ. ID. No. 7), indicated in the expression units of table 4 were replaced by the vascular promoter AT5G59880 (SEQ. ID. No. 13). As in example 1, the resulting sequences were synthesized and assembled in the order listed, they were sequenced to verify that no mutations were inserted in the assembly process and finally cloned into the vector of Genscript company, containing carbenicillin antibiotic resistance.

As in example 1, the resulting recombinant plasmids were transformed into competent cells of *Agrobacterium tumefaciens* and *A. rhizogenes.* We used carbenicillin antibiotic, which is the synthetic version of ampicillin and is more stable. Plasmid DNA was extracted from the resistant bacteria, and the presence of the recombinant plasmid was verified on agarose gel.

**Table 4**

| **Expression Unit** | **Assembly and sequence order** | **SEQ. ID. No.** |
|---|---|---|
| CsPP16-Lysozyme | 1. T-DNA left border of *A. tumefaciens* | 1 |
| | 2. Promoter 35S short version of the cauliflower mosaic virus | 2 |
| | 3. Gene encoding the protein 16K of the phloem of *Citrus aurantifolia* orthologous in citrus or protein CsPP16 | 3 |
| | 4. Flexible polylinker | 4 |
| | 5. Human lysozyme with codon usage in citrus | 5 |
| | 6. Terminator NOS and the T-DNA right border of *A. tumefaciens* | 6 |
| CsPP16-Defensin | 1. T-DNA left border of *A. tumefaciens* | 1 |
| | 2. Promoter 35S short version of the cauliflower mosaic virus | 2 |
| | 3. Gene encoding the protein 16K of the phloem of *Citrus aurantifolia* orthologous in citrus or protein CsPP16 | 3 |
| | 4. Flexible polylinker | 4 |
| | 5. Human defensin with codon usage in citrus | 8 |
| | 6. Terminator NOS and T-DNA right border *A. tumefaciens* | 6 |
| CsPP16-Magainin | 1. T-DNA left border of *A. tumefaciens* | 1 |
| | 2. Promoter 35S short version of the cauliflower mosaic virus | 2 |
| | 3. Gene encoding the protein 16K of the phloem of *Citrus aurantifolia* orthologous in citrus or protein CsPP16 | 3 |
| | 4. Flexible polylinker | 4 |
| | 5. Magainin with codon usage in citrus | 9 |
| | 6. Terminator NOS and T-DNA right border of *A. tumefaciens* | 6 |
| CsPP16-Cecropin | 1. T-DNA left border of *A. tumefaciens* | 1 |
| | 2. Promoter 35S short version of the cauliflower mosaic virus | 2 |
| | 3. Gene encoding the protein 16K of the phloem of *Citrus aurantifolia* orthologous in citrus or protein CsPP16 | 3 |
| | 4. Flexible polylinker | 4 |
| | 5. Cecropin with codon usage in citrus | 10 |
| | 6. Terminator NOS and T-DNA right border of *A. tumefaciens* | 6 |
| CsPP16-Sarcotoxin | 1. T-DNA left border of *A. tumefaciens* | 1 |
| | 2. Promoter 35S short version of the cauliflower mosaic virus | 2 |
| | 3. Gene encoding the protein 16K of the phloem of *Citrus aurantifolia* orthologous in citrus or protein CsPP16 | 3 |
| | 4. Flexible polylinker | 4 |
| | 5. Sarcotoxin with codon usage in citrus | 11 |
| | 6. Terminator NOS and T-DNA right border of *A. tumefaciens* | 6 |
| CsPP16-Indolicidin | 1. T-DNA left border of *A. tumefaciens* | 1 |
| | 2. Promoter 35S short version of the cauliflower mosaic virus | 2 |
| | 3. Gene encoding the protein 16K of the phloem of *Citrus aurantifolia* orthologous in citrus or protein CsPP16 | 3 |
| | 4. Flexible polylinker | 4 |
| | 5. Indolicidin with codon usage in citrus | 12 |
| | 6. Terminator NOS and T-DNA right border of *A. tumefaciens* | 6 |

To verify the presence of the gene of interest with antimicrobial activity inserted into the vector or on the plant thereby transformed, specific oligonucleotide primers were used for PCR amplification; for example, in the case of lysozyme, the forward oligonucleotide 3'-AGGTTTTTCGAAAGATGCGAACTTGCTAGAA-5' (SEQ. ID. No. 14), and the reverse 3'-AAACACCGCAACCTTGAACATATTGTCTGC-5' (SEQ. ID. No. 15); and in the case of defensin, the forward oligonucleotide 3'- ATGAGAGTTCTTTATCTTCTTTTCAGCTTC-5' (SEQ. ID. No. 16), and the reverse 3'- ACTTCTTCTTGCAGCATCTTGTACCTGGAA-5' (SEQ. ID. No. 17).

**Example 3. Transient expression of the constructs of the invention in Mexican lime plants infected with HLB, using** ***Agrobacterium.** Agrobacterium* was grown in LB medium at 30°C under constant stirring to reach an O.D. of 0.4 (600 nm). Acetosyringone was added to the cells to a final concentration of 140 micromolar, incubating for two additional hours with the inducer. Bacteria were harvested by centrifugation and resuspended in transformation medium (figure 7).

The method of inoculation of adult plants included removing the lignified bark with sandpaper, exposing the green photosynthetic tissue. A pre moisturized swab with the recombinant bacteria containing some of the expression vectors described in examples 1 and/or 2 was placed on the tissue, and resuspended in transformation medium. The swab was temporarily fixed around the photosynthetic tissue uncovered with plastic and the plant was covered with a plastic bag and maintained in high humidity for two days. After the incubation, the bag and the swab were removed. Plants continued to be watered to maintain adequate water content favoring the transfer of T-DNA to the treated area.

Materials of citrus created using patrons of volkamerian lime grafted into Mexican lime and/or Persian lime were used in the assays. The plants showed symptoms associated with HLB, and their bacterial load of *Candidatus Liberibacter* was confirmed by real-time PCR. The experimental design performed is shown in table 5.

**Table 5. Experimental design used to transform mature lime trees.**

| **Treatment** | **No. of Plants** | **No. of Inoculations** |
|---|---|---|
| Plants diseased with Lysozyme | 5 | 4 |
| Plants diseased with Defensin | 5 | 4 |
| Plants diseased with Lysozyme and Defensin | 5 | 4 |
| Plants diseased with transgene control (GUS as a reporter gene) | 5 | 4 |
| Plants diseased with water inoculation | 5 | 4 |
| Healthy plants | 5 | 4 |
| Healthy plants with water | 1 | 4 |

The photosynthetic capacity of the plants was measured using IRGA system at constant radiation, taking ten measurements per plant.

The plants were inoculated by scraping the woody stem tissue with a scalpel, wherein a swab containing the bacterial solution was deposited on the exposed region. The swab was covered with plastic, and the whole plant was covered with a large plastic bag to maintain high humidity and favor the generation of transformed tissue (figure 8). The plants were irrigated with water only to avoid masking the symptoms of HLB. The analysis of the plants was performed at 60 and 120 days after inoculation.

The analysis of the plants was performed at 60 days after inoculation. We analyzed the effect of the expression of lysozyme, defensin, and the combination thereof, plus healthy controls, diseased controls, and a control that consisted of using the GUS reporter gene, an indicator of the gene transformation event.

The inoculation of the plants with these treatments implies the integration of the T-DNA containing the gene of interest in the exposed tissue. Generally, the avirulent *Agrobacterium* species produce a small tumor in the applied area, evidence of the transformation. The plants treated were analyzed for the presence of transformed tissue, finding small tumor areas as shown in figure 9.

**Example 4. Measurement of photosynthesis and leaf area of treated plants according to the present invention.** Ten measurements of each plant were taken, which were recorded and averaged with Excel software; wherein the µmol units CO₂/cm².S⁻¹ . The figure 10 shows the graph of the values obtained. The CO₂ fixation value of diseased plants was less than those of the treatments. Note that the value obtained in diseased plants treated with GUS showed frank heterotrophy. Plants treated with the constructs of the invention that include antimicrobial peptides have greater photosynthetic capacity, which can be attributed to an improvement in the physiological status of the plant two months after inoculation. At that time (60 days), the resulting photosynthesis after the defensin treatment was less than that obtained with lysozyme. However, at 210 days both treatments showed photosynthesis values similar to those of healthy plants.

At the same time, the number of new shoots in these plants and their size was quantified. As shown in figure 11, the GUS control plants (diseased without antimicrobial treatment) showed fewer new shoots, wherein the treatments with CsPP16-defensin and defensin/lysozyme showed a greater number. Treatments comprised applying to the bacteria transformed with the vector containing CsPP16-defensin a second treatment with CsPP16-lysozyme, and a third treatment with a mixture of bacteria containing CsPP16-defensin and CsPP16-lysozyme. We consistently observed that the treatments with defensin produce a greater number of shoots, which could be because this gene acted more rapidly in controlling the bacteria *Candidatus Liberibacter.* After 210 days, the number of shoots in the treatments was statistically similar. Also, figure 12 shows in a graph the size of the new shoots; wherein the treatments with CsPP16-defensin and CsPP16-lysozyme showed a greater size. As described, the differences at 60 days were no longer observed as time progressed in the treatments. Note that during this period, the plants were not fertilized to evidencing HLB symptoms, which are more pronounced when the plants suffer nutritional stress. Figure 12 shows the appearance of a shoot obtained with the treatment lysozyme/defensin according to the invention; the shoot appears without symptoms and with leaf areas similar to healthy plants.

After 60 days of inoculation, the new shoots showed no further symptom associated to HLB, compared to the preexisting shoots, where despite the inoculation the symptoms characteristic of HLB remained. This was for us an indication that possibly owed to the excessive accumulation of callose in the phloem, the free movement of proteins is blocked through this element, preventing it from reaching those tissues. This is consistent with the photosynthesis analysis shown earlier, where heterotrophy in those tissues is evident. The new shoots to not presenting any visible symptom of the disease, led us to conclude that it is owed to the treatments with antimicrobials according to the invention; figure 13 shows the appearance of plants treated according to the invention after 100 days of treatment. It also shows the comparison of control plants and those treated with antimicrobials, wherein a clear improvement in symptoms in the shoots of the treated plants was observed.

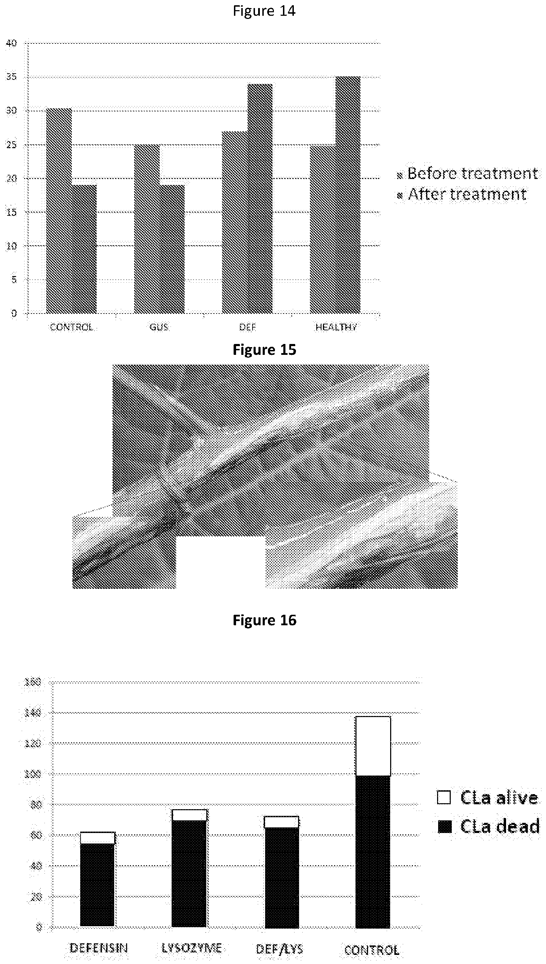

The measurement of leaf areas was conducted before and after 60 days of treatment in the new shoots of each treatment, analyzing 10 leaves per each plant. The leaf areas of each plant were averaged and analyzed by the graph shown in figure 14, wherein we can observe an increase in the leaf area of the plants with the antimicrobial treatment similar to the leaf areas of the healthy plant, contrary to what happens with the control plants where we observe a decrease in the leaf areas, possibly owed to disease progression. Also, in plants inoculated with *Agrobacterium,* we observed in the stem the development of small tumors in the inoculation area, which indicated the presence of hyper producer zones of the antimicrobials as shown in figure 15.

**Example 5. Measuring antimicrobial content in the transformed plants.** We obtained plant samples consisting of preexisting and new leaves. The plants were processed according to testing procedures established by the Servicio Nacional de Sanidad, Inocuidad y Calidad Agroalimentaria of SAGARPA, Mexico.

Briefly, the midrib of the leaf was dissected and cut into small sections with the aid of a disposable razor. The tissue was weighed (100 mg) and processed in two ways, one for obtaining total DNA using a commercial purification kit, and the other for the treatment with Mono ethidium azide (MEA) and subsequent purification of DNA, to discriminate if the DNA used as template was present in live or dead bacteria.

Figure 16 shows the results of this analysis. As can be seen, the number of live bacteria decreased in the treatment, although it was not destroyed completely. It should be noted that this transient expression assay expresses in discrete regions of the plant the antimicrobial used, and the amount produced cannot be controlled. The graph shows the total size of the bar as indication of the total bacterial detection, and in blue color the number of dead bacteria, while the red color shows the number of live bacteria. Generally, treatments have up to one sixth of the load in the diseased control plants. This result is very promising considering that these plants are chimeras producers of antimicrobials; by using methods of gene transformation for obtaining plants fully transformed plants in all the tissues it is possible to achieve a resistance to the disease close to 100%.

The graph in figure 17 shows the relation of live/dead bacterial load after 60 days of treatment, wherein we can observe that the number of live bacteria in the plants that were treated with the antimicrobial is up to 4 times lower than the control plants, wherein the number of both dead and live bacteria outnumber by far the plants treated.

**Example 6. Stable expression test in plants of Mexican lime infected with HLB.** Stable expression of lysozyme in Mexican lime is accomplished by transforming lime explants and once regenerated; they are grafted into lime patterns, which allow obtaining mature transgenic plants expressing the protein of interest in a short time.

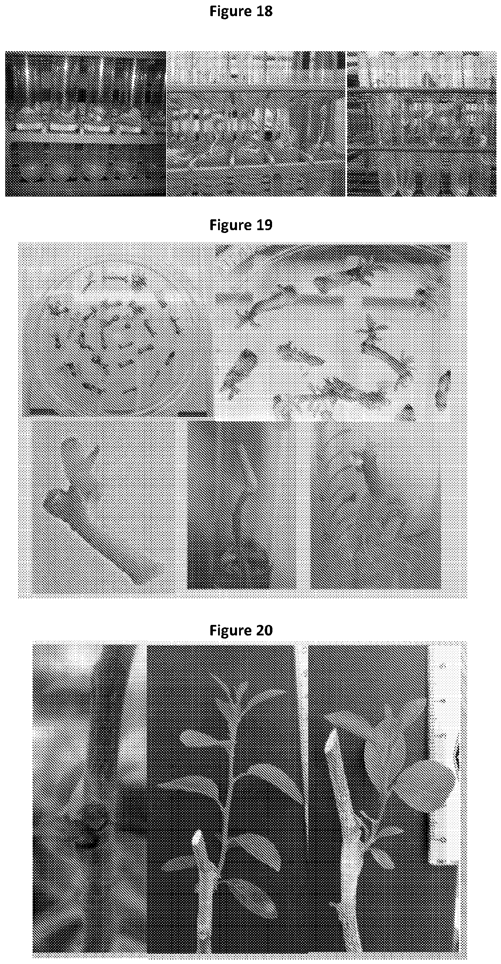

*In vitro* germination of Mexican lime seeds was conducted in assay tubes containing sterile germination medium as shown in figure 18. In the composition of the medium are mineral elements, water, hormones, organic substances, vitamins, and sucrose, which promote the germination of the lime seeds. The seeds were previously sterilized with a 30% chlorine solution for half hour, followed by three washes with sterile water. Sowing of seeds was performed under sterile conditions. Once planted the seeds were incubated at room temperature in the dark for approximately 2 to 3 weeks. After this time, the shoot of the cotyledon was observed and again incubated for 1 week at room temperature under photoperiod (16 hours of light and 8 hours of dark); this, to allow the activation of the photosynthetic activity of the plant.

The plants thus obtained were then used to obtain explants, which were transformed by *Agrobacterium tumefaciens* and *Agrobacterium rhizogenes* containing the CsPP16-Lysozyme construct.

For the transformation of lime explants with *A. tumefaciens* and *A. rhizogenes,* these bacteria were first grown in LB medium at 30°C under stirring to reach an O.D. of 0.4 (600 nm). The cells were added acetosyringone to a final concentration of 140 micromolar and incubated for two additional hours with the inducer. Bacteria were harvested by centrifugation and resuspended in transformation medium. The stems of the limes germinated *in vitro* were cut to an approximate length of 1 cm with a knife previously dipped in transformation medium under sterile conditions. The excess fluid of the cut explants was removed by using sterile absorbent paper. The explants were transferred to co-cultivation medium (CM), where they were incubated at room temperature for 1-2 days in the dark. After this time, the explants were re-transferred to regeneration medium (SRM) and incubated at room temperature in the dark for 4 weeks. Subsequently, the explants were incubated for another 4 to 6 weeks at room temperature under photoperiod (16 hours of light and 8 hours of dark) until complete regeneration. Explants were changed to fresh SRM every 4 weeks or incase of contamination. Figures 7, 8, and 19 show the process of transformation and regeneration of Mexican lime explants.

For propagating the transformed explants in different patterns of Mexican lime, these were grafted into patterns of volkamerian lime, citrange troyer, or rough Schaub.

The grafts in the above patterns were making a cut in the form of "V" at 15 cm from the base of the pattern, and carefully placing within this cut the transformed explant in its entirety. The graft was secured with parafilm. The plant was watered with water only, and the graft was covered with a plastic bag to maintain high relative humidity and allow the regeneration. Sixty days after graft implantation, the lysozyme expression was analyzed by PCR endpoint and qRT-PCR using oligonucleotides such as those described in examples 1 and 2.

The composition of the culture media used for the transformation of Mexican lime was: LB medium (950 mL distilled water; 10 g tryptone; 5 g yeast extract; 10 g NaCl with pH 7 adjusted with NaOH 1N (this solution is adjusted to a final volume of 1 liter with deionized water and sterilized); medium for germinating lime seeds (1 liter of MS medium, wherein 30 g sucrose and 2 g rite gel are dissolved); MC co-culture medium (1 liter of MS medium, wherein 2 mg indole-3-acetic acid, 1 mg 2-isopentyl-adenine, 2 mg 1,4-dichlorophenoxyacetic acid, and 8 g agar are dissolved; the pH at 5.2 is adjusted with NaOH 1N and sterilized); SRM regeneration medium (1 liter MS medium where 3 mg 6-benzylaminopurine and 10 g agar are dissolved; the pH at 5.2 is adjusted with NaOH 1N and sterilized; once the medium is warm, 250 mg/L of vancomicyn and 500 mg/L of cefotaxime antibiotics were added).

**Example 7. Antimicrobial transmission through grafting.** Genetic transformations of Persian lime and Mexican lime citrus were performed on rootstock patterns of *C. volkameriana* and *C. macrophylla* sour orange. In these assays, the antimicrobial rootstocks containing defensin, lysozyme, and a mixture thereof was transformed, each one of them translationally fused with the protein CsPP16, which is the protein of systemic protein of 16KDa citrus. The transformation was performed by exposing the stem tissue to an abrasive, and the tissue was subsequently contacted with a solution of *A. tumefaciens* or *A. rhizogenes,* containing the genetic constructs described (figure 8). The plants used were grafted into infected shoots of HLB, which presented the characteristic symptoms of the disease.

The plants were analyzed for the presence of the protein in grafts and of their agronomic characteristics such as plant height, photosynthesis, leaf area, and quantification of the bacteria.

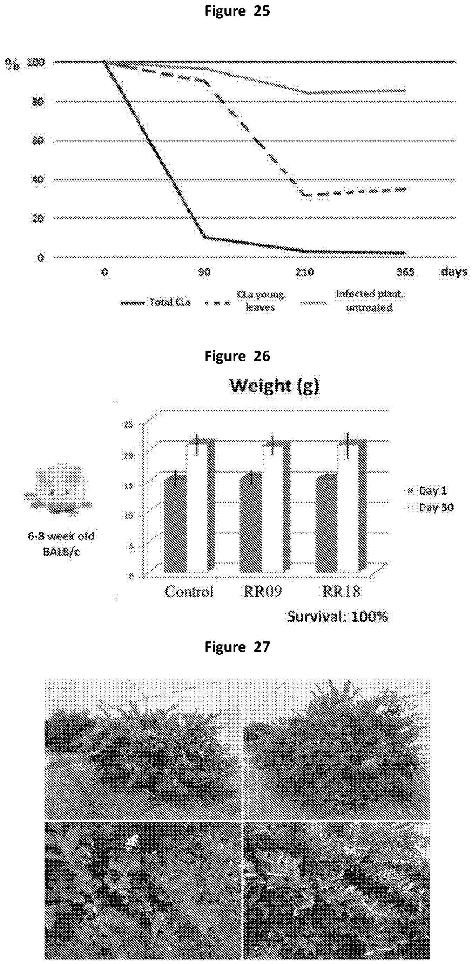

Previously treated plants were monitored for one year, finding that new shoots and fruits were produced with a reduced quantity of the bacteria regarding the initial accounts. Old and new leaves were monitored; finding the highest concentration in the old leaves and not in the new shoots. It should be noted that the diffuse mottling symptom did not disappear from mature leaves. This fact is interpreted as an inability to mobilize leaf starch polymer generated by this mottling symptom. Figure 25 shows the behavior at one year of the analysis of plants diseased with HLB and which were transformed in their rootstocks of volkameriana and macrophylla, and branches of the graft with the constructs described.

Samples of leaves with symptoms, such as asymptomatic systemic leaves were collected, their DNA extracted, and the presence of CLa and COX endogenous gene was quantified by real-time PCR techniques. A variant of this method for detecting live bacteria in the tissue analyzed was to conduct the EMA PCR technique. Interestingly, it is shown that the live bacteria decreased around 70% and are confined to old tissues. In contrast, young tissues show very low values or below the detection limit of the technique. Figure 25 shows the behavior of the presence of live bacteria in the control plants, ranging between 100 and 87% of CLa bacteria. Photosynthesis was comparable between healthy plants and that of young leaves. It is noted that the plants were able to produce spherical fruit with radial symmetry, characteristic of plants with a healthy vascular tissue (figure 21).

These evidences allow us to assert that the use of genetically transformed patterns with proteins of supra cellular nature are sufficient to confer resistance to the bacteria causing HLB or others present in the vascular tissues of plants.

The fruits produced by infected plants treated with antimicrobials according to the present invention showed commercial grade and were used to conduct toxicity testing.

The fruits obtained from plants treated according to the present invention showed the characteristic symmetry of healthy fruits (figure 21), showing a cross section of a lime diseased with HLB, presenting asymmetric locules, while a fruit from a diseased plant that was treated with antimicrobials according to the present invention shows a symmetry comparable to the fruit of a healthy plant.

**Example 8. Acute toxicity test in mice.** 10 BALB-c mice per treatment were fed with normal diet and instead of simple water they were given lime water with 2% juice of the fruits from plants treated according to the present invention (RR09 Defensin and RR18 Lysozyme). The mice were weighed at the start of the experiment. After 30 days of the experiment, they were weighed again and a sample of peripheral blood was obtained by tail vein puncture.

As can be seen in figure 26, there was no animal mortality in treatments performed (lysozyme and defensin, and in the control group). Similarly, mice showed statistically similar weights between treatments at the end of the test.

The blood collected from mice was analyzed by hematologic micro biometry. When comparing the values obtained from both the control group and the treatments, these were in the ranges proposed as normal values (table 6). As can be seen, the hematologic biometry of mice treated was similar to that of mice in the control group, obtaining values in the range reported as normal for BALB-c mice of 10-14 weeks. Note that at the end of the acute toxicity test 100% survival was obtained both in treated and in control animals.

**Table 6. HLB lime. Hematologic biometry.**

| **Component** | **Reference ranges** | | **Average** | | |
|---|---|---|---|---|---|
| | **Minimum** | **Maximum** | **Defensin** | **Lysozyme** | **Control** |
| Erythrocytes/µl | 7 | 1.2 x 10⁷ | 2 x 10⁶ | 1.9 x 10⁶ | 1.6 x 10⁶ |
| Platelets/µl | 630,000 | 1,550,000 | 665,000 | 749,125 | 447,000 |
| Leukocytes/µl | 6,000 | 15,000 | 11,975 | 11,378 | 11,394 |
| Lymphocytes/µl | 3,400 | 13,950 | 8,242 | 8,914 | 8,830 |
| Segmented/µl | 480 | 7,200 | 1,922 | 1,926 | 1,922 |
| Neutrophils/µl | 480 | 7,200 | 1,689 | 2,072 | 1,990 |
| Monocytes/µl | 60 | 1,050 | 570 | 203 | 146 |
| Eosynophils/µl | 120 | 1,500 | 499 | 189 | 334 |
| V.G.M. u3 | 57 | 93 | 78 | 78 | 77 |
| Band/µl | 35 | 45 | 57 | 56 | 58 |
| V.G.M. u3 | 45 | 55 | 51 | 49 | 51 |
| Hemoglobin g/dl | 12.2 | 16.2 | 17.2 | 14.6 | 12.9 |
| H.G.M. pg | 11 | 12.7 | 10.9 | 12.7 | 11.3 |
| Total plasma proteins | 6.2 | 7.4 | 7.5 | 7.8 | 7.3 |

**Example 9. Transgenes are not detected in pollen of treated plants.** Pollen and flowers of plants treated with antimicrobials according to the present invention were collected, for which total DNA was extracted from pollen and flowers collected from treated and control plants. The endogenous gene was amplified, not the encoding antimicrobial, demonstrating that the plants produce pollen and wildflowers. This experimental evidence shows that the transgene cannot be inadvertently dispersed to a potential receiver plant; similarly, the transgene will not be present in the fruit, which allows obtaining a non-genetically modified fruit and therefore, for example not subject to the law of Genetic Modified Organisms (GMO) bio safety governing current Mexican law.

### Example 10. Tests in other citrus.

**a) Genetic transformation of Persian lime, Valencia orange, grapefruit, and mandarine grafted into volkamerikana.** Aside from the tests in Mexican lime with thorns, we performed the induction of the expression of antimicrobials according to the present invention in Persian lime, Valencia orange, grapefruit, and mandarine, all of them grafted both into *Citrus macrophylla* and *C*. *volkameriana* (table 7). 100 plants for each variety were treated, of which 25 were treated with lysozyme 25 with defensin, and 25 with a mixture thereof. The remaining 25 were used as controls of the experiment and were inoculated with water.

**Table 7**

| **Treatment** | **Persian Lime** | **Valencia Orange** | **Grapefruit** | **Mandarine** |
|---|---|---|---|---|
| Lysozyme | 25 | 25 | 25 | 25 |
| Defensin | 25 | 25 | 25 | 25 |
| Lys/Def | 25 | 25 | 25 | 25 |
| Control | 25 | 25 | 25 | 25 |

One year old trees with 1.5 m average size and safety certificate from the Vivarium Francisco Villa in Tamaulipas, Mexico, were inoculated as described above for Mexican lime. The plants were in high humidity chamber for 3 days and then transplanted to micro tunnels prepared with an anti-aphid mesh.

The meshes were removed to allow the entry of psyllids infected with CLa, as the biocontainment area is located in a HLB endemic region in Tecoman, Colima, Mexico. The presence of psyllids infected was monitored and the possible infection of treatment and control plants in the referred cultivars. After 8 months of planting, no symptoms nor the positive detection of bacteria were observed when using molecular methods for the detection of CLa in treated plants, but not in the control plants, which were infected and showed symptoms associated with the presence of HLB.

In conjunction with the quantifications of the bacteria, their growth and photosynthetic capacity were monitored, wherein no significant differences were found between treatment and control plants. One year after treatments, bacteria levels are very low or below the detection limits of the technique used (real-time PCR with Taqman probes).

**b) Genetic transformation of productive Mexican lime trees grafted in *Citrus volkameriana.*** 24 productive 3-year old trees were selected in the HLB endemic zone in Tecoman, Colima, Mexico, and the presence of the bacteria causing HLB was quantified on them, producing high titers in symptomatic leaf samples with diffused mottles. The plants were subjected to induction treatment of antimicrobial expression as described above, with a distribution shown in table 8.

**Table 8**

| **Tree** | **Treatment** | **Color** | **Tree** | **Treatment** | **Color** |
|---|---|---|---|---|---|
| 1 | Lysozyme | Red | 13 | Control | White |
| 2 | Defensin | Green | 14 | Lys/Def | Blue |
| 3 | Lys/Def | Blue | 15 | Defensin | Green |
| 4 | Control | White | 16 | Lysozyme | Red |
| 5 | Lysozyme | Red | 17 | Control | White |
| 6 | Defensin | Green | 18 | Lys/Def | Blue |
| 7 | Lys/Def | Blue | 19 | Defensin | Green |
| 8 | Control | White | 20 | Lysozyme | Red |
| 9 | Lysozyme | Red | 21 | Control | White |
| 10 | Defensin | Green | 22 | Lys/Def | Blue |
| 11 | Lys/Def | Blue | 23 | Defensin | Green |
| 12 | Control | White | 24 | Lysozyme | Red |

The trees of three years after planting had a mean size of 3 m. The plants were biocontained in tunnels framed with aluminum and anti-aphid mesh. Two entrances to the property restricted access and greenhouses had locked doors.

The trees were marked by placing a ribbon with the color of the treatment (table 8) and an aluminum label with the tree number in their trunk.

As seen in figure 27, adult trees of Mexican lime under confinement at 3 months generated ramifications; wherein some of the first leaves showed symptoms. However, the extent of the symptoms was confined to that area. Also, it was observed that the new branches did not have symptoms of the disease 4 months after the treatment according to the present invention. The evaluation at 9 months after the treatment shows symmetrical trees with shoots and normal flowering. New shoots have fewer symptoms such as diffuse mottling or lack thereof. Their photosynthetic rate is high, indicating a good physiological condition.

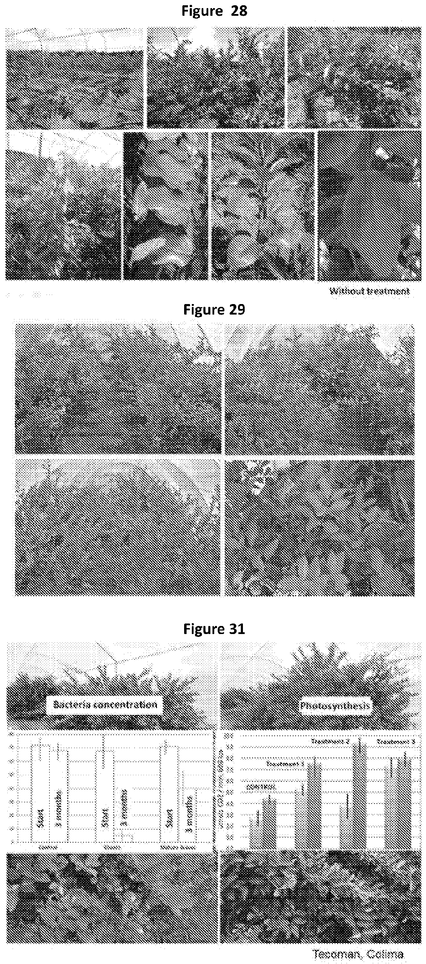

The appearance of the plants grown in micro tunnels after 12 months (Mexican lime) and 6 months treatment (Persian lime, orange, grapefruit, and mandarine) according to the present invention, showed growth, and the apparition of ramifications without HLB symptoms (figure 28). Moreover, plants grown under bio containment, produce shoots free from CLa when receiving the antimicrobial treatment according to the present invention (figure 29).

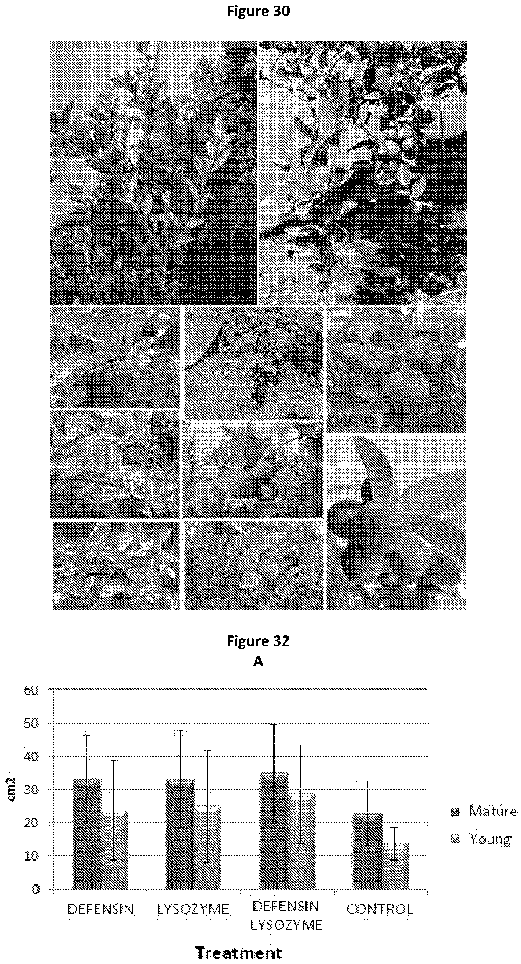

Measuring photosynthetic parameters showed that the plants treated according to the present invention have photosynthesis values similar to the vivarium plants grown in absence of the psyllid. Also, the treated plants produce flowers and fruit filling of commercial grade (figure 30). The appearance of the plants treated with antimicrobials at five months of being in contact with the psyllid is normal, they do not show HLB symptoms in preexisting leaves and new shoots. In contrast, control plants without antimicrobial treatment show in the same batch the presence of symptoms in their leaves and have, on average, fewer ramifications. Figure 28 (bottom right panel) shows the symptoms of an untreated plant, presenting the characteristic symptoms of the disease.

Figure 21 shows significant differences between the fruits of a healthy lime, which present carpels with radial symmetry (left panel), while the fruit produced by a diseased tree shows an asymmetric development of the carpels and the apparition of two acrocentric chromosomes (central panel). As a result of the treatment of lime trees initially diseased and subsequently treated according to the present invention, the fruits obtained are alike to those from a healthy tree (right panel).

Moreover, the bacterial content at three months was estimated, showing that the bacteria are present both in mature leaves and new shoots. In contrast, although the bacterium is detected in mature leaves existing from the start of the treatment, new leaves have significantly reduced in number. Regarding photosynthesis, untreated control plants show lower photosynthesis in contrast to the three treatments tested (treatment 1: defensin, treatment 2: lysozyme and treatment 3: mixture of defensin with lysozyme) (figure 31).

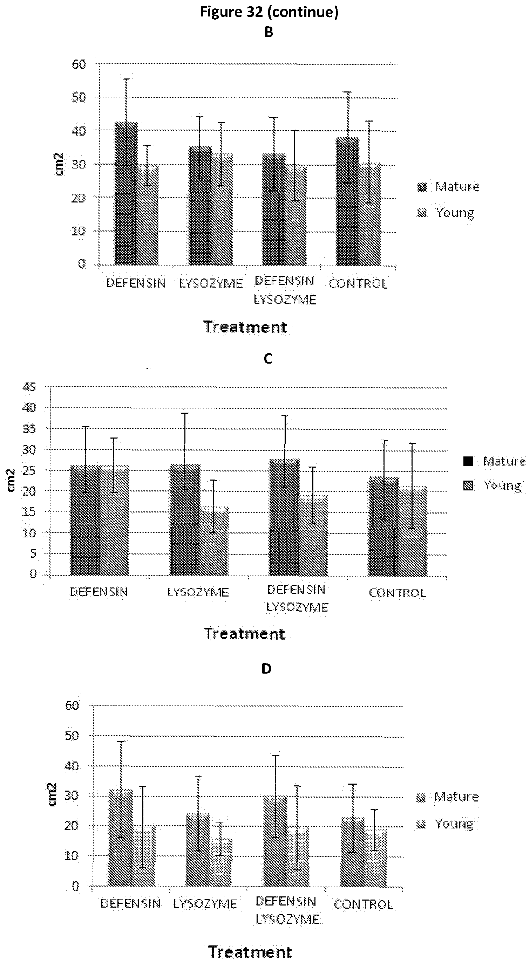

Regarding the Persian lime, mandarine, grapefruit, and orange trees, plants were evaluated for their agronomic parameters, finding no significant differences six months after being treated in relation to their leaf area. However, the CLa content is different in the control plants, which already show the presence of bacteria and symptoms characteristics of HLB (figure 32).

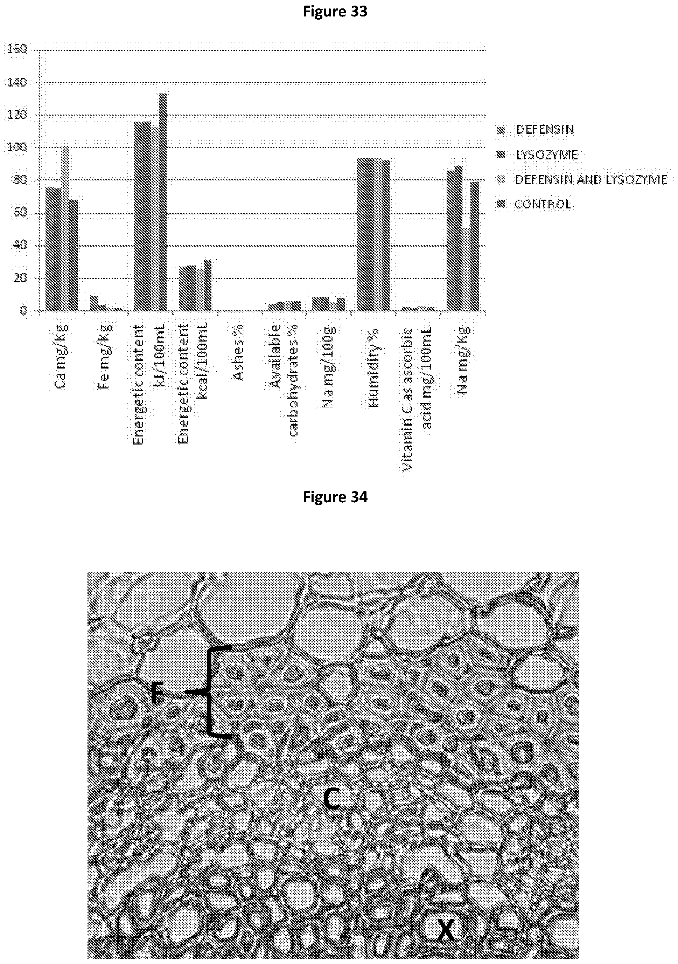

**Example 11. Bromatological analysis of lime-juice.** Lime fruits derivate from trees treated with defensin, lysozyme, and a mixture thereof, in addition to limes of control trees, were collected. Juice was extracted using a juicer and summoned for determining bromatological parameters according to Mexican and FDA norms for juices. The results are shown in figure 33. As observed, the concentrations of calcium, iron, energy content, ash, available carbohydrates, sodium, vitamin C (ascorbic acid), and moisture are similar among fruit juices subjected to treatment of the invention and the control juice, demonstrating substantial equivalence among them.

**Example 12. Immunodetection of defensin protein in transgenic lime and evidence for the presence of transgenes.** Genetically modified lime plants expressing the antimicrobial defensin or lysozyme according to the present invention were processed for inclusion in paraffin and perform immunodetection tests. Semi-thin sections were performed, deparaffinized, and hydrated to subsequently incubate them with anti-human-beta-defensin antibodies. The first antibody generated in goat was immunodetected with goat anti-rabbit antibody coupled to alkaline phosphatase and developed with BCIP and NBT, which generate a blue-violet color, indicating the presence of the protein immunodetected. As shown in figure 34, defensin was localized in the phloem of the transformed plant.

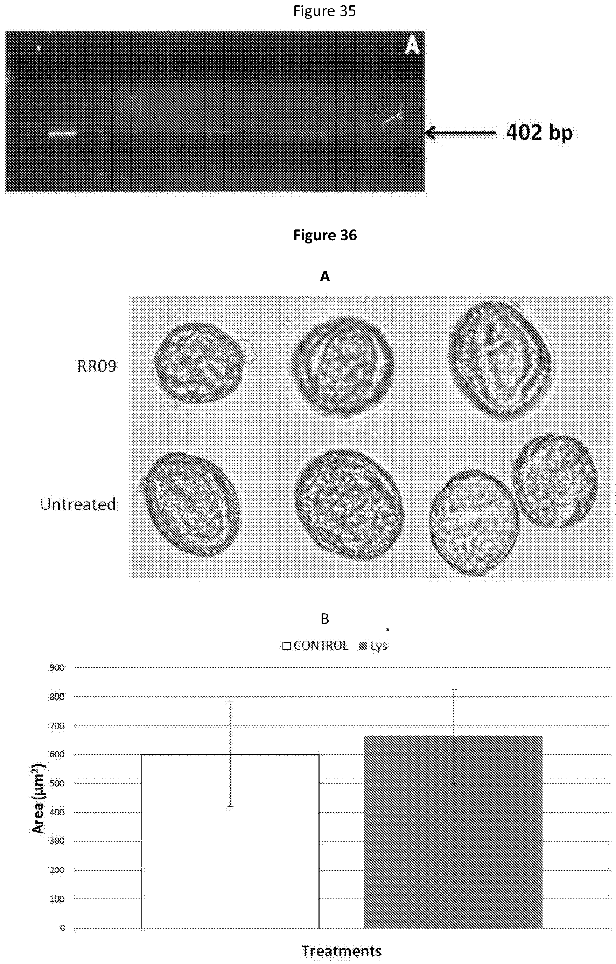

Also and by end-point PCR, in the transformed plants according to the present invention we detected the genes encoding the antimicrobials above described (figure 35), which constituting a molecular evidence of the presence of said transgenes in the genetically modified plants via tissue culture. Using PCR techniques, the insertion sites in the genome of GM citrus have been identified.

**Example 13. Morphology of pollen obtained from treated plants.** Pollen from plants treated according to the present invention, which was photographed and measured to assess its substantial equivalence, were collected. Figure 36 (panel A) shows representative images of the pollen, and we can see that its morphological characteristics are similar to control plants.

The average area of pollen was obtained, and there are no statistically significant differences among them (figure 36, panel B). The results show no alterations in the area or in the morphology of the pollen obtained from treatment plants.

As can be seen from the above, according to the present invention it is possible to obtain genetically modified plants expressing proteins with antimicrobial activity, such as the citrus tested, which have the ability to protect themselves from infection caused by *Candidatus Liberibacter asiaticus;* e.g., Mexican lime, Persian lime, orange, mandarine and grapefruit. Also, the present invention allows obtaining rootstocks (patterns) of genetically modified citrus expressing antimicrobial proteins. The experimentally tested patterns are *Citrus volkameriana* and *Citrus macrophylla,* which are resistant to citrus tristeza virus. Genetically modified rootstocks can mobilize antimicrobials via the vascular tissue to the grafts of plants susceptible to the infection, such as Mexican lime, Persian lime, orange, mandarine and grapefruit, as demonstrated experimentally, and thus, providing protection to grafts. The rootstocks have the advantage of producing conventional fruits without the presence of genes encoding antimicrobials, and the fruits are not subject, for example, to the Mexican law of bio safety use of genetically modified organisms. This also implies a great safety in their consumption and production.

According to the present invention, for healthy plants produced in certified vivariums, the transient expression of antimicrobials is performed via inoculation of *Agrobacterium tumefaciens* containing in their T-DNA the expression units of antimicrobials. This inoculation can be performed on healthy plants as a preventive method. At the time of planting endemic areas, such as HLB, plants will not infect or will show very low levels of the bacteria, thus allowing the generation of conventional fruits, which will not be subject, for example, to the Mexican law of bio safety use of genetically modified organisms. Also, the product can be used in new plantings or replace diseased plants.

For productive trees diseased with HLB, the transient expression of antimicrobials is performed according to the present invention via the inoculation of *Agrobacterium tumefaciens* containing the antimicrobial expression units in its T-DNA. This inoculation can be performed on healthy plants as a preventive method. At the time of planting endemic areas, such as HLB, plants will not infect or will show very low levels of the bacteria, thus allowing the generation of conventional fruits, which will not be subject, for example, to the Mexican law of bio safety use of genetically modified organisms. Also, the product can be used in new plantings or replace diseased plants.

### References.

- Bové J.M. (2006). Huanglongbing: A destructive, newly-emerging, century-old disease of citrus. Journal of Plant Pathology, 88 (1), 7-37.
- Cervera, M., et.al. 1998. Genetic transformation and regeneration of mature tissue of woody fruit plants bypassing the juvenile stage. Transgenic Res. 7, 51-59.
- Cervera, M., et.al. 2004. Genetic transformation of mature citrus plants. Methods Mol. Biol. 286, 177-187.
- Corbesier L., et.al. 2007. FT protein movement contributes to long-distance signaling in floral induction of Arabidopsis. Science 316: 1030-1033.
- Hartung J.S., et.al. 2011. Comparison of the 'Ca. Liberibacter asiaticus' genome adapted for an intracellular lifestyle with other members of the Rhizobiales. PLoS One. 6(8):e23289.
- Koh E.J., et.al. 2011. Callose deposition in the phloem plasmodesmata and inhibition of phloem transport in citrus leaves infected with "Candidatus Liberibacter asiaticus". Protoplasma PMID: 21874517.
- Lin H., et.al. 2011. The complete genome sequence of 'Candidatus Liberibacter solanacearum', the bacterium associated with potato zebra chip disease. PLoS One 6: e19135.
- Lin M.K., Belanger H., Lee Y.J., Varkonyi-Gasic E., Taoka K., Miura E., Xoconostle-Cazares B., Gendler K., Jorgensen R.A., Phinney B., Lough T.J., Lucas W.J. 2007. FLOWERING LOCUS T protein may act as the long-distance florigenic signal in the cucurbits. Plant Cell 19: 1488-1506.
- Lough T.J., et.al. 2006. Integrative plant biology: role of phloem long-distance macromolecular trafficking. Annu Rev Plant Biol 57:203-232.
- Masschalck B., et.al. 2001. Inactivation of gram-negative bacteria by lysozyme, denatured lysozyme, and lysozyme-derived peptides under high hydrostatic pressure. Appl Environ Microbiol. 67:339-344.
- Provencher L.M., et.al. 2001. Sucrose export defective1 encodes a novel protein implicated in chloroplast-to-nucleus signaling. Plant Cell 13:1127-1141.
- Ruiz-Medrano R., Xoconostle-Cazares B., Ham B.K., Li G., Lucas W.J. 2011. Vascular expression in Arabidopsis is predicted by the frequency of CT/GA-rich repeats in gene promoters. Plant J 67:130-144.
- Ruiz-Medrano R., Xoconostle-Cazares B., Kragler F. 2004. The plasmodesmatal transport pathway for homeotic proteins, silencing signals and viruses. Curr Opin Plant Biol 7: 641-650.
- Ruiz-Medrano R., Xoconostle-Cazares B., Lucas W.J. 2001. The phloem as a conduit for inter-organ communication. Curr Opin Plant Biol. 4:202-209.
- Strategic Planning for the Florida Citrus Industry: Addressing Citrus Greening. 2010. Committee on the Strategic Planning for the Florida Citrus Industry: Addressing Citrus Greening Disease (Huanglongbing); National Research Council of the National Academies USA. ISBN 978-0-309-15207-5.
- Vaa Bel A.J., et.al. 2004. Pathogen-induced resistance and alarm signals in the phloem. Mol Plant Pathol 5:495-504.
- Xoconostle-Cazares B., Xiang Y., Ruiz-Medrano R., Wang H.L., Monzer J., Yoo B.C., McFarland K.C., Franceschi V.R., Lucas W.J. 1999. Plant paralog to viral movement protein that potentiates transport of mRNA into the phloem. Science. 283:94-98.

## Claims

1. A plant or parts thereof, resistant to infections caused by microorganisms which are restricted to the phloem, wherein the plant incorporates in its genome a chimeric gene encoding a fusion protein that acts as a transporter inside the vascular tissue of the plant and has an antimicrobial activity, wherein said chimeric gene comprises a gene that codifies the protein CsPP16 linked to a gene that codifies for a protein with antimicrobial activity, wherein both genes are linked through a flexible linker, wherein said gene that codifies the protein CsPP16 has the sequence of SEQ. ID. No. 3, said flexible linker has the sequence of SEQ. ID. No. 4 and said gene that codifies the protein with antimicrobial activity, has a sequence selected from the group comprising SEQ. ID. No. 5, SEQ. ID. No. 8, SEQ. ID. No. 9, SEQ. ID. No. 10, SEQ. ID. No. 11, SEQ. ID. No. 12.

2. The plant according to claim 1, wherein said microorganism is a bacteria.

3. The plant according to claim 2, wherein said bacteria is selected form the group comprising *Candidatus Liberobacter asiaticus, Candidatus Liberobacter africanus, Candidatus Liberobacter americanus* and *Candidatus Liberibacter solanacearum.*

4. The plant according to claim 1, wherein said plant is selected form the group comprising limes *(Citrus limon),* oranges *(Citrus sinensis),* mandarine oranges *(Citrus reticulata),* grapefruits (*Citrus reticulata x Citrus paradisi*), tangerines (*Citrus paradisi*), *Citrus limonia, Citrus limettioides, Aeglopsis chevalieri Swingle, Atalantia missionis Oliver, Balsamocitrus dawei Stapf., Calodendrum capensis Thunb, Catharanthus roseus (L.) Citroncirus webberi, Citrus amblycarpa Ochse, Citrus aurantiifolia Swingle, Citrus aurantium L., Citrus depressa Hayata, Citrus grandis (L.) Osbeck, Citrus hassaku Hort. ex Tanaka, Citrus hystrix DC., Citrus ichangensis Swingle, Citrus jambhiri Lushington, Citrus junos Sieb. ex Tanaka, Citrus kabuchi Hort. ex Tanaka, Citrus limon (L.) Burm., Citrus x limonia Osbeck, Citrus x nobilis Lour. "Ortanique", Citrus maxima (pomelo*/*shaddock), Citrus x nobilis Lour., Citrus oto Hort. ex Tanaka, Citrus x paradisi Macfad., Citrus reticulata Blanco, Citrus sinensis (L.) Osbeck, Citrus sunki Hort. ex Tanaka, Citrus unshiu (Mack.) Marc, Clausena indica Oliver, Clausena lansium (Lour.) Skeels, Cuscuta australis R. Br. (Convolvulaceae, Cuscutaceae), Fortunella spp., Limonia acidissima L., Microcitrus australasica (F.J. Muell.) Swingle, Murraya koenigii (L.), Murraya paniculata (L.) Jack, Poncirus trifoliata (L.) Raf., Swinglea glutinosa (Blanco) Merr., Toddalia lanceolata Lam, Triphasia trifolia (Burm. f.) P. Wilson, Citrus indica Tanaka, Citrus limetta Risso* and *Citrus macroptera Montrons.*

5. A recombinant vector comprising an expression unit that expresses a fusion protein that acts as a transporter inside the vascular tissue of a plant and has an antimicrobial activity wherein the expression unit comprises a gene that codifies the protein CsPP16 linked to a gene that codifies for a protein with antimicrobial activity, wherein both genes are linked through a flexible linker wherein said gene that codifies the protein CsPP16 has the sequence of SEQ. ID. No. 3, said flexible linker has the sequence of SEQ. ID. No. 4 and said gene that codified for a protein with antimicrobial activity, has a sequence selected from the group comprising SEQ. ID. No. 5, SEQ. ID. No. 8, SEQ. ID. No. 9, SEQ. ID. No. 10, SEQ. ID. No. 11, SEQ. ID. No. 12.

6. A host cell, comprising the recombinant vector of claim 5, wherein said host cell is capable of transfer of genetic material to a plant.

7. The host cell according to claim 6, wherein said cell is *Agrobacterium.*

8. A method for obtaining a plant or parts thereof, resistant to infections caused by microorganisms which are restricted to the phloem according to claim 1, comprising the steps of:
a) conjugating to a host cell capable of transfer of genetic material to a plant, the recombinant vector of claim 5,
b) inoculating a plant susceptible to infections caused by microorganisms which are restricted to the phloem, with the host cell obtained in step a), and
c) verifying the acquisition of the vector and its expression, its stability in the transformed plants verifying the resistance acquired.

9. A method for treating a plant or parts thereof infected by microorganisms which are restricted to the phloem, comprising the steps of:
a) inoculating the infected plant with a host cell capable to transfer genetic material to a plant, wherein such host cell has been previously conjugated with the recombinant vector of claim 5, and
b) verifying in the infected plant the acquisition of the vector and its expression, its stability in the infected transformed plants and verifying the resistance acquired to the infection.

10. The method according to claim 8 or 9, wherein in the step a) the host cell capable of transferring genetic material to a plant is *Agrobacterium.*

11. The method according to claim 10, wherein said *Agrobacterium* is selected from the group comprising *Agrobacterium tumefaciens* and *Agrobacterium rhizogenes.*

12. The method according to claim 8 or 9, wherein said plant is selected form the group comprising limes *(Citrus limon),* oranges *(Citrus sinensis),* mandarine oranges *(Citrus reticulata),* grapefruits *(Citrus reticulata x Citrus paradisi),* tangerines *(Citrus paradisi), Citrus limonia, Citrus limettioides, Aeglopsis chevalieri Swingle, Atalantia missionis Oliver, Balsamocitrus dawei Stapf., Calodendrum capensis Thunb, Catharanthus roseus (L.) Citroncirus webberi, Citrus amblycarpa Ochse, Citrus aurantiifolia Swingle, Citrus aurantium L., Citrus depressa Hayata, Citrus grandis (L.) Osbeck, Citrus hassaku Hort. ex Tanaka, Citrus hystrix DC., Citrus ichangensis Swingle, Citrus jambhiri Lushington, Citrus junos Sieb. ex Tanaka, Citrus kabuchi Hort. ex Tanaka, Citrus limon (L.) Burm., Citrus x limonia Osbeck, Citrus x nobilis Lour. "Ortanique", Citrus maxima (pomelo*/*shaddock), Citrus x nobilis Lour., Citrus oto Hort. ex Tanaka, Citrus x paradisi Macfad., Citrus reticulata Blanco, Citrus sinensis (L.) Osbeck, Citrus sunki Hort. ex Tanaka, Citrus unshiu (Mack.) Marc, Clausena indica Oliver, Clausena lansium (Lour.) Skeels, Cuscuta australis R. Br. (Convolvulaceae, Cuscutaceae), Fortunella spp., Limonia acidissima L., Microcitrus australasica (F.J. Muell.) Swingle, Murraya koenigii (L.), Murraya paniculata (L.) Jack, Poncirus trifoliata (L.) Raf., Swinglea glutinosa (Blanco) Merr., Toddalia lanceolata Lam, Triphasia trifolia (Burm. f.) P. Wilson, Citrus indica Tanaka, Citrus limetta Risso* and *Citrus macroptera Montrons.*

13. The method according to claim 9, wherein said infectious microorganism is selected from the group comprising *Candidatus Liberobacter asiaticus, Candidatus Liberobacter africanus, Candidatus Liberobacter americanus* and *Candidatus Liberibacter solanacearum.*

14. The method according to claim 13, wherein said infectious microorganism causes in the plant a disease selected from the group comprising Zebra chip disease and Huanglongbing disease.

15. A chimeric gene, encoding a fusion protein that acts as a transporter inside the vascular tissue of a plant and has an antimicrobial activity, comprising a gene that codifies the protein CsPP16 linked to a gene that codifies for a protein with antimicrobial activity, wherein both genes are linked through a flexible linker wherein said gene that codifies the protein CsPP16 has the sequence of SEQ. ID. No. 3 the flexible linker has the sequence of SEQ. ID. No. 4 and the gene that codified for a protein with antimicrobial activity, has a sequence selected from the group comprising SEQ. ID. No. 5, SEQ. ID. No. 8, SEQ. ID. No. 9, SEQ. ID. No. 10, SEQ. ID. No. 11, SEQ. ID. No. 12.

16. A protein that acts as a transporter inside the vascular tissue of the plant and has an antimicrobial activity, and wherein said protein is codified by the chimeric gene of claim 15.

17. A composition for treating a plant or parts thereof infected by microorganisms which are restricted to the phloem, comprising the host cell according to claim 6 in an agronomically compatible vehicle.

## Patentansprüche

1. Pflanze oder Teile davon, die gegen Infektionen resistent sind, die durch Mikroorganismen hervorgerufen werden, die sich auf das Phloem beschränken, wobei die Pflanze ein chimäres Gen in ihr Genom aufnimmt, das ein Fusionsprotein kodiert, das als ein Transporter innerhalb des vaskulären Gewebes der Pflanze fungiert und über eine antimikrobielle Aktivität verfügt, wobei das chimäre Gen ein Gen umfasst, das das Protein CsPP16 kodiert, das mit einem Gen verknüpft ist, das ein Protein mit antimikrobieller Aktivität kodiert, wobei beide Gene durch einen flexiblen Linker verknüpft sind, wobei das Gen, das das Protein CsPP16 kodiert, über die Sequenz von SEQ. ID. No. 3 verfügt, der flexible Linker über die Sequenz von SEQ. ID. No. 4 verfügt und das Gen, das das Protein mit antimikrobieller Aktivität kodiert, über eine Sequenz aus der folgenden Gruppe verfügt: SEQ. ID. No. 5, SEQ. ID. No. 8, SEQ. ID. No. 9, SEQ. ID. No. 10, SEQ. ID. No. 11, SEQ. ID. No. 12.

2. Pflanze gemäß Anspruch 1, wobei der Mikroorganismus ein Bakterium ist.

3. Pflanze gemäß Anspruch 2, wobei das Bakterium aus der folgenden Gruppe ausgewählt wird: *Candidatus Liberobacter asiaticus, Candidatus Liberobacter africanus, Candidatus Liberobacter americanus und Candidatus Liberibacter solanacearum.*

4. Pflanze gemäß Anspruch 1, wobei die Pflanze aus der folgenden Gruppe ausgewählt wird: Limetten *(Citrus limon),* Orangen *(Citrus sinensis),* Mandarinen *(Citrus reticulata),* Grapefruits *(Citrus reticulata x Citrus paradisi),* Tangerinen *(Citrus paradisi), Citrus limonia, Citrus limettioides, Aeglopsis chevalieri Swingle, Atalantia missionis Oliver, Balsamocitrus dawei Stapf., Calodendrum capensis Thunb, Catharanthus roseus (L.) Citroncirus webberi, Citrus amblycarpa Ochse, Citrus aurantiifolia Swingle, Citrus aurantium L., Citrus depressa Hayata, Citrus grandis (L.) Osbeck, Citrus hassaku Hort. ex Tanaka, Citrus hystrix DC., Citrus ichangensis Swingle, Citrus jambhiri Lushington, Citrus junos Sieb. ex Tanaka, Citrus kabuchi Hort. ex Tanaka, Citrus limon (L.) Burm., Citrus x limonia Osbeck, Citrus x nobilis Lour. "Ortanique", Citrus maxima (pomelo*/*shaddock), Citrus x nobilis Lour., Citrus oto Hort. ex Tanaka, Citrus x paradisi Macfad., Citrus reticulata Blanco, Citrus sinensis (L.) Osbeck, Citrus sunki Hort. ex Tanaka, Citrus unshiu (Mack.) Marc, Clausena indica Oliver, Clausena lansium (Lour.) Skeels, Cuscuta australis R. Br. (Convolvulaceae, Cuscutaceae), Fortunella spp., Limonia acidissima L., Microcitrus australasica (F.J. Muell.) Swingle, Murraya koenigii (L.), Murraya paniculata (L.) Jack, Poncirus trifoliata (L.) Raf., Swinglea glutinosa (Blanco) Merr., Toddalia lanceolata Lam, Triphasia trifolia (Burm. f.) P. Wilson, Citrus indica Tanaka, Citrus limetta Risso* und *Citrus macroptera Montrons.*

5. Rekombinanter Vektor, der eine Expressionseinheit umfasst, die ein Fusionsprotein exprimiert, das als ein Transporter innerhalb des vaskulären Gewebes einer Pflanze fungiert und über eine antimikrobielle Aktivität verfügt, wobei die Expressionseinheit ein Gen umfasst, das das Protein CsPP16 kodiert, das mit einem Gen verknüpft ist, das ein Protein mit antimikrobieller Aktivität kodiert, wobei beide Gene durch einen flexiblen Linker verknüpft sind, wobei das Gen, das das Protein CsPP16 kodiert, über die Sequenz von SEQ. ID. No. 3 verfügt, der flexible Linker über die Sequenz von SEQ. ID. No. 4 verfügt und das Gen, das das Protein mit antimikrobieller Aktivität kodiert, über eine Sequenz aus der folgenden Gruppe verfügt: SEQ. ID. No. 5, SEQ. ID. No. 8, SEQ. ID. No. 9, SEQ. ID. No. 10, SEQ. ID. No. 11, SEQ. ID. No. 12.

6. Wirtszelle, die den rekombinanten Vektor gemäß Anspruch 5 umfasst, wobei die Wirtszelle in der Lage ist genetisches Material auf eine Pflanze zu übertragen.

7. Wirtszelle gemäß Anspruch 6, wobei die Zelle *Agrobakterium* ist.

8. Verfahren zum Erhalten einer Pflanze oder Teilen davon, die gegen Infektionen resistent sind, die durch Mikroorganismen hervorgerufen werden, die auf das Phloem beschränkt sind, gemäß Anspruch 1, das die folgenden Schritte umfasst:
a) Konjugieren des rekombinanten Vektors gemäß Anspruch 5 mit einer Wirtszelle, die in der Lage ist genetisches Material auf eine Pflanze zu übertragen.
b) Impfen einer Pflanze, die gegenüber Infektionen anfällig ist, die durch Mikroorganismen verursacht werden, die sich auf das Phloem beschränken, mit der in Schritt a) erhaltenen Wirtszelle, und
c) Prüfen der Übernahme des Vektors und seiner Expression, seiner Stabilität in den transformierten Pflanzen und Prüfen der erworbenen Resistenz.

9. Verfahren zum Behandeln einer Pflanze oder Teilen davon, die durch Mikroorganismen infiziert sind, die sich auf das Phloem beschränken, das die folgenden Schritte umfasst:
a) Impfen der infizierten Pflanze mit einer Wirtszelle, die in der Lage ist genetisches Material auf eine Pflanze zu übertragen, wobei eine solche Wirtszelle zuvor mit dem rekombinanten Vektor gemäß Anspruch 5 konjugiert worden ist, und
b) Prüfen der Übernahme des Vektors und seiner Expression in der infizierten Pflanze, seiner Stabilität in den infizierten transformierten Pflanzen und Prüfen der erworbenen Resistenz gegenüber der Infektion.

10. Verfahren gemäß Anspruch 8 oder 9, wobei in dem Schritt a) die Wirtszelle, die in der Lage ist genetisches Material auf eine Pflanze zu übertragen, *Agrobakterium* ist.

11. Verfahren gemäß Anspruch 10, wobei das *Agrobakterium* aus der folgenden Gruppe ausgewählt wird: *Agrobacterium tumefaciens* und *Agrobacterium rhizogenes.*

12. Verfahren gemäß Anspruch 8 oder 9, wobei die Pflanze aus der folgenden Gruppe ausgewählt wird: Limetten *(Citrus limon),* Orangen *(Citrus sinensis),* Mandarinen *(Citrus reticulata),* Grapefruits *(Citrus reticulata x Citrus paradisi),* Tangerinen *(Citrus paradisi), Citrus limonia, Citrus limettioides, Aeglopsis chevalieri Swingle, Atalantia missionis Oliver, Balsamocitrus dawei Stapf., Calodendrum capensis Thunb, Catharanthus roseus (L.) Citroncirus webberi, Citrus amblycarpa Ochse, Citrus aurantiifolia Swingle, Citrus aurantium L., Citrus depressa Hayata, Citrus grandis (L.) Osbeck, Citrus hassaku Hort. ex Tanaka, Citrus hystrix DC., Citrus ichangensis Swingle, Citrus jambhiri Lushington, Citrus junos Sieb. ex Tanaka, Citrus kabuchi Hort. ex Tanaka, Citrus limon (L.) Burm., Citrus x limonia Osbeck, Citrus x nobilis Lour. "Ortanique", Citrus maxima (pomelo*/*shaddock), Citrus x nobilis Lour., Citrus oto Hort. ex Tanaka, Citrus x paradisi Macfad., Citrus reticulata Blanco, Citrus sinensis (L.) Osbeck, Citrus sunki Hort. ex Tanaka, Citrus unshiu (Mack.) Marc, Clausena indica Oliver, Clausena lansium (Lour.) Skeels, Cuscuta australis R. Br. (Convolvulaceae, Cuscutaceae), Fortunella spp., Limonia acidissima L., Microcitrus australasica (F.J. Muell.) Swingle, Murraya koenigii (L.), Murraya paniculata (L.) Jack, Poncirus trifoliata (L.) Raf., Swinglea glutinosa (Blanco) Merr., Toddalia lanceolata Lam, Triphasia trifolia (Burm. f.) P. Wilson, Citrus indica Tanaka, Citrus limetta Risso* und *Citrus macroptera Montrons.*

13. Verfahren gemäß Anspruch 9, wobei der infektiöse Mikroorganismus aus der folgenden Gruppe ausgewählt wird: *Candidatus Liberobacter asiaticus, Candidatus Liberobacter africanus, Cancidatus Liberobacter americanus* und *Candidatus Leberibacter solanacearum.*

14. Verfahren gemäß Anspruch 13, wobei der infektiöse Mikroorganismus in der Pflanze eine Krankheit verursacht, die aus der folgenden Gruppe ausgewählt wird: Zebra-Chip-Krankheit und Huanglongbing (Citrus Greening).

15. Chimäres Gen, das ein Fusionsprotein kodiert, das als ein Transporter innerhalb des vaskulären Gewebes einer Pflanze fungiert und über eine antimikrobielle Aktivität verfügt, das ein Gen umfasst, das das Protein CsPP16 kodiert, das mit einem Gen verknüpft ist, das ein Protein mit antimikrobieller Aktivität kodiert, wobei beide Gene durch einen flexiblen Linker verknüpft sind, wobei das Gen, das das Protein CsPP16 kodiert, über die Sequenz von SEQ. ID. No. 3 verfügt, der flexible Linker über die Sequenz von SEQ. ID. No. 4 verfügt und das Gen, das das Protein mit antimikrobieller Aktivität kodiert, über eine Sequenz aus der folgenden Gruppe verfügt: SEQ. ID. No. 5, SEQ. ID. No. 8, SEQ. ID. No. 9, SEQ. ID. No. 10, SEQ. ID. No. 11, SEQ. ID. No. 12.

16. Protein, das als ein Transporter innerhalb des vaskulären Gewebes der Pflanze fungiert und über eine antimikrobielle Aktivität verfügt, und wobei das Protein durch das chimäre Gen gemäß Anspruch 15 kodiert wird.

17. Zusammensetzung zum Behandeln einer Pflanze oder Teilen davon, die durch Mikroorganismen infiziert sind, die sich auf das Phloem beschränken, die die Wirtszelle gemäß Anspruch 6 in einem agronomisch verträglichen Mittel umfasst.

## Revendications

1. Plante ou parties de celle-ci, résistante(s) aux infections provoquées par des micro-organismes qui se limitent au phloème, la plante intégrant dans son génome un gène chimérique codant pour une protéine de fusion qui agit comme un transporteur à l'intérieur du tissu vasculaire de la plante et a une activité antimicrobienne, ledit gène chimérique comprenant un gène qui code pour la protéine CsPP16 liée à un gène qui code pour une protéine ayant une activité antimicrobienne, les deux gènes étant liés par le biais d'un lieur flexible, ledit gène qui code pour la protéine CsPP16 ayant la séquence de SEQ ID No. 3, ledit lieur flexible ayant la SEQ ID No. 4, et ledit gène qui code pour la protéine ayant une activité antimicrobienne ayant une séquence choisie dans le groupe comprenant la SEQ ID No. 5, la SEQ ID No. 8, la SEQ ID No. 9, la SEQ ID No. 10, la SEQ ID No. 11, la SEQ ID No. 12.

2. Plante selon la revendication 1, dans laquelle ledit micro-organisme est une bactérie.

3. Plante selon la revendication 2, dans laquelle ladite bactérie est choisie dans le groupe comprenant *Candidatus Liberobacter asiaticus, Candidatus Liberobacter africanus, Candidatus Liberobacter americanus* et *Candidatus Liberibacter solanacearum.*

4. Plante selon la revendication 1, ladite plante étant choisie dans le groupe comprenant les citrons *(Citrus limon),* les oranges *(Citrus sinensis),* les mandarines *(Citrus reticulata),* les pamplemousses *(Citrus reticulata x Citrus paradisi),* les tangerines *(Citrus paradisi), Citrus limonia, Citrus limettioides, Aeglopsis chevalieri Swingle, Atalantia missionis Oliver, Balsamocitrus dawei Stapf., Calodendrum capensis Thunb, Catharanthus roseus (L.) Citroncirus webberi, Citrus amblycarpa Ochse, Citrus aurantiifolia Swingle, Citrus aurantium L., Citrus depressa Hayata, Citrus grandis (L.) Osbeck, Citrus hassaku Hort. ex Tanaka, Citrus hystrix DC., Citrus ichangensis Swingle, Citrus jambhiri Lushington, Citrus junos Sieb. ex Tanaka, Citrus kabuchi Hort. ex Tanaka, Citrus limon (L.) Burm., Citrus x limonia Osbeck, Citrus x nobilis Lour. « Ortanique », Citrus maxima (pomelo*/*shaddock), Citrus x nobilis Lour., Citrus oto Hort. ex Tanaka, Citrus x paradisi Macfad., Citrus reticulata Blanco, Citrus sinensis (L.) Osbeck, Citrus sunki Hort. ex Tanaka, Citrus unshiu (Mack.) Marc, Clausena indica Oliver, Clausena lansium (Lour.) Skeels, Cuscuta australis R. Br. (Convolvulaceae, Cuscutaceae), Fortunella spp., Limonia acidissima L., Microcitrus australasica (F.J. Muell.) Swingle, Murraya koenigii (L.), Murraya paniculata (L.) Jack, Poncirus trifoliata (L.) Raf., Swinglea glutinosa (Blanco) Merr, Toddalia lanceolata Lam, Triphasia trifolia (Burm. f.) P. Wilson, Citrus indica Tanaka, Citrus limetta Risso* et *Citrus macroptera Montrons.*

5. Vecteur recombinant comprenant une unité d'expression qui exprime une protéine de fusion qui agit comme un transporteur à l'intérieur du tissu vasculaire d'une plante et a une activité antimicrobienne, l'unité d'expression comprenant un gène qui code pour la protéine CsPP16 liée à un gène qui code pour une protéine ayant une activité antimicrobienne, les deux gènes étant liés par le biais d'un lieur flexible, ledit gène qui code pour la protéine CsPP16 ayant la séquence de SEQ ID No. 3, ledit lieur flexible ayant la SEQ ID No. 4, et ledit gène qui code pour une protéine ayant une activité antimicrobienne ayant une séquence choisie dans le groupe comprenant la SEQ ID No. 5, la SEQ ID No. 8, la SEQ ID No. 9, la SEQ ID No. 10, la SEQ ID No. 11, la SEQ ID No. 12.

6. Cellule hôte, comprenant le vecteur recombinant selon la revendication 5, ladite cellule hôte étant capable de transférer un matériel génétique à une plante.

7. Cellule hôte selon la revendication 6, dans laquelle ladite cellule est *Agrobacterium.*

8. Procédé d'obtention d'une plante ou de parties de celle-ci, résistante(s) aux infections provoquées par des micro-organismes qui se limitent au phloème selon la revendication 1, comprenant les étapes consistant à :
a) conjuguer une cellule hôte capable de transférer du matériel génétique à une plante, le vecteur recombinant de la revendication 5,
b) inoculer une plante sensible aux infections provoquées par des micro-organismes qui se limitent au phloème avec la cellule hôte obtenue à l'étape a), et
c) vérifier l'acquisition du vecteur et son expression, sa stabilité dans les plantes transformées et vérifier la résistance acquise.

9. Procédé de traitement d'une plante ou de parties de celle-ci, infectée(s) par des micro-organismes qui se limitent au phloème, comprenant les étapes consistant à :
a) inoculer la plante infectée avec une cellule hôte capable de transférer du matériel génétique à une plante, une telle cellule hôte ayant été au préalable conjuguée avec le vecteur recombinant de la revendication 5, et
b) vérifier dans la plante infectée l'acquisition du vecteur et son expression, sa stabilité dans les plantes transformées infectées et vérifier la résistance acquise à l'infection.

10. Procédé selon la revendication 8 ou 9, dans lequel, à l'étape a), la cellule hôte capable de transférer du matériel génétique à une plante est *Agrobacterium.*

11. Procédé selon la revendication 10, dans lequel ledit *Agrobacterium* est choisi dans le groupe comprenant *Agrobacterium tumefaciens et Agrobacterium rhizogenes.*

12. Procédé selon la revendication 8 ou 9, dans lequel ladite plante est choisie dans le groupe comprenant les citrons *(Citrus limon),* les oranges *(Citrus sinensis),* les mandarines *(Citrus reticulata),* les pamplemousses *(Citrus reticulata x Citrus paradisi),* les tangerines *(Citrus paradisi), Citrus limonia, Citrus limettioides, Aeglopsis chevalieri Swingle, Atalantia missionis Oliver, Balsamocitrus dawei Stapf., Calodendrum capensis Thunb, Catharanthus roseus (L.) Citroncirus webberi, Citrus amblycarpa Ochse, Citrus aurantiifolia Swingle, Citrus aurantium L., Citrus depressa Hayata, Citrus grandis (L.) Osbeck, Citrus hassaku Hort. ex Tanaka, Citrus hystrix DC., Citrus ichangensis Swingle, Citrus jambhiri Lushington, Citrus junos Sieb. ex Tanaka, Citrus kabuchi Hort. ex Tanaka, Citrus limon (L.) Burm., Citrus x limonia Osbeck, Citrus x nobilis Lour. « Ortanique », Citrus maxima (pomelo*/*shaddock), Citrus x nobilis Lour., Citrus oto Hort. ex Tanaka, Citrus x paradisi Macfad., Citrus reticulata Blanco, Citrus sinensis (L.) Osbeck, Citrus sunki Hort. ex Tanaka, Citrus unshiu (Mack.) Marc, Clausena indica Oliver, Clausena lansium (Lour.) Skeels, Cuscuta australis R. Br. (Convolvulaceae, Cuscutaceae), Fortunella spp., Limonia acidissima L., Microcitrus australasica (F.J. Muell.) Swingle, Murraya koenigii (L.), Murraya paniculata (L.) Jack, Poncirus trifoliata (L.) Raf., Swinglea glutinosa (Blanco) Merr, Toddalia lanceolata Lam, Triphasia trifolia (Burm. f.) P. Wilson, Citrus indica Tanaka, Citrus limetta Risso* et *Citrus macroptera Montrons.*

13. Procédé selon la revendication 9, dans lequel ledit micro-organisme infectieux est choisi dans le groupe comprenant *Candidatus Liberobacter asiaticus, Candidatus Liberobacter africanus, Candidatus Liberobacter americanus* et *Candidatus Liberibacter solanacearum.*

14. Procédé selon la revendication 13, dans lequel ledit micro-organisme infectieux provoque dans la plante une maladie choisie dans le groupe comprenant la maladie de la chips zébrée et la maladie de dragon jaune.

15. Gène chimérique, codant pour une protéine de fusion qui agit comme un transporteur à l'intérieur du tissu vasculaire d'une plante et possède une activité antimicrobienne, comprenant un gène qui code pour la protéine CsPP16 liée à un gène qui code pour une protéine ayant une activité antimicrobienne, les deux gènes étant liés par le biais d'un lieur flexible, ledit gène qui code pour la protéine CsPP16 ayant la séquence de SEQ ID No. 3, ledit lieur flexible ayant la SEQ ID No. 4, et ledit gène qui code pour la protéine ayant une activité antimicrobienne ayant une séquence choisie dans le groupe comprenant la SEQ ID No. 5, la SEQ ID No. 8, la SEQ ID No. 9, la SEQ ID No. 10, la SEQ ID No. 11, la SEQ ID No. 12.

16. Protéine qui agit comme un transporteur à l'intérieur du tissu vasculaire de la plante et a une activité antimicrobienne et ladite protéine étant codée par le gène chimérique de la revendication 15.

17. Composition pour le traitement d'une plante ou des parties de celle-ci infectée(s) par les micro-organismes qui se limitent au phloème, comprenant la cellule hôte selon la revendication 6 dans un véhicule agronomiquement compatible.


### SEQUENCE LISTING

<110> Centro de Investigación y de Estudios Avanzados del
   Instituto Politecnico Nacional
   Secretaria de Agricultura, Ganadería, Desarrollo Rural,
   Pesca y Alimentación
<120> Plants resistant to pathogenic microorganisms growing in vascular tissues
<130> Cinvestavl7
<160> 17
<170> PatentIn version 3.5
<210> 1
   <211> 162
   <212> DNA
   <213> Agrobacterium tumefaciens
<220>
   <221> misc_feature
   <223> T-DNA left border of A. tumefaciens
<400> 1
   cggttaaaac caccccagta cattaaaaac gtccgcaatg tgttattaag ttgtctaagc       60
   gtcaatttgt ttacaccaca atatatcctg ccaccagcca gccaacagct ccccgaccgg      120
   cagctcggca caaaatcacc actcgataca ggcagcccat ag                        162
<210> 2
   <211> 524
   <212> DNA
   <213> Cauliflower mosaic virus
<220>
   <221> misc_feature
   <223> Promoter 35S short version of the cauliflower mosaic virus
<400> 2
   tcaaagattc aaatagagga cctaacagaa ctcgccgtaa agactggcga acagttcata        60
   cagagtctct tacgactcaa tgacaagaag aaaatcttcg tcaacatggt ggagcacgac       120
   acacttgtct actccaaaaa tatcaaagat acagtctcag aagaccaaag ggcaattgag       180
   acttttcaac aaagggtaat atccggaaac ctcctcggat tccattgccc agctatctgt       240
   cactttattg tgaagatagt ggaaaaggaa ggtggctcct acaaatgcca tcattgcgat       300
   aaaggaaagg ccatcgttga agatgcctct gccgacagtg gtcccaaaga tggaccccca       360
   cccacgagga gcatcgtgga aaaagaagac gttccaacca cgtcttcaaa gcaagtggat       420
   tgatgtgata tctccactga cgtaagggat gacgcacaat cccactatcc ttcgcaagac       480
   ccttcctcta tataaggaag ttcatttcat ttggagagaa cacg                        524
<210> 3
   <211> 489
   <212> DNA
   <213> Citrus aurantifolia
<220>
   <221> misc_feature
   <223> Gene encoding the protein 16K of the phloem of Citrus aurantifolia orthologous in citrus or protein CsPP16
<400> 3
   atggggatgg gaatgatgga ggtccatttg attagtggta aaggacttca agcccacgac        60
   cctcttaata aaccaattga cccttatgct gagatcaact tcaagggcca agaacgcatg       120
   agcaaagttg ctaaaaatgc aggaccaaat ccattatggg atgagaaatt taaattccta       180
   gccgagtacc ctgggagcgg tggtgacttc catatcttat tcaaggtcat ggaccatgac       240
   gctatagatg gtgatgatta cattggcgat gtcaaaatag atgtcaagaa tttattagcg       300
   gaaggagtta gaaaaggaaa gtcggagatg ccgcctagaa tgtaccatgt cttagcacac       360
   aaaatccact tcaagggaga aattgaagtt ggagtgtcct tcaagctgca aggcggcggg       420
   ggatgtggag gatgttaccc atgggaaaac cttagcggtg gtggtggtag cggtggtggt       480
   ggtagcggt                                                               489
<210> 4
   <211> 36
   <212> DNA
   <213> Artificial Sequence
<220>
   <223> Sintetic
<220>
   <221> misc_feature
   <223> Flexible polylinker
<400> 4
   ggtggtggta gcggtggtgg tggtagcgct gctgct 36
<210> 5
   <211> 402
   <212> DNA
   <213> Artificial Sequence
<220>
   <223> sintetic
<220>
   <221> misc_feature
   <223> Human lysozyme with codon usage in citrus
<400> 5
   aaggttttcg aaagatgcga acttgctaga acacttaaga gacttggtat ggatggttat      60
   agaggtatta gccttgctaa ttggatgtgc cttgctaagt gggaaagcgg ttataataca     120
   agagctacaa attataatga tagaagcaca gattatggta ttttccaaat taatagcaga     180
   tattggagat gcaatgatgg taagacacca ggtgctgtta atgcttgcca tcttagctgc     240
   agcgctcttc ttcaagataa tattgctgat aatattgctg atgctgttgc ttgcgctaag     300
   agagttgtta gagatccaca aggtattaga gcttgggttg cttggagaaa tagatgccaa     360
   aatagagatt gcagacaata tgttcaaggt tgcggtgttt ag                        402
<210> 6
   <211> 419
   <212> DNA
   <213> Agrobacterium tumefaciens
<220>
   <221> misc_feature
   <223> Terminator NOS and the T-DNA right border of A. tumefaciens
<400> 6
   gatcgttcaa acatttggca ataaagtttc ttaagattga atcctgttgc cggtcttgcg      60
   atgattatca tataatttct gttgaattac gttaagcatg taataattaa catgtaatgc     120
   atgacgttat ttatgagatg ggtttttatg attagagtcc cgcaattata catttaatac     180
   gcgatagaaa acaaaatata gcgcgcaaac taggataaat tatcgcgcgc ggtgtcatct     240
   atgttactag atctttggat ctcctgtggt tggcaaatgg acgaacggat aaaccttttc     300
   acgccctttt aaatatccga ttattctaat aaacgctctt ttctcttagg tttacccgcc     360
   aatatatcct gtcaaacact gatagtttaa actgaaggcg ggaaacgaca atctgctag      419
<210> 7
   <211> 833
   <212> DNA
   <213> Cauliflower mosaic virus
<220>
   <221> misc_feature
   <223> Promoter 35S large version of the cauliflower mosaic virus
<400> 7
   attagccttt tcaatttcag aaagaatgct aacccacaga tggttagaga ggcttacgca      60
   gcaggtctca tcaagacgat ctacccgagc aataatctcc aggaaatcaa ataccttccc     120
   aagaaggtta aagatgcagt caaaagattc aggactaact gcatcaagaa cacagagaaa     180
   gatatatttc tcaagatcag aagtactatt ccagtatgga cgattcaagg cttgcttcac     240
   aaaccaaggc aagtaataga gattggagtc tctaaaaagg tagttcccac tgaatcaaag     300
   gccatggagt caaagattca aatagaggac ctaacagaac tcgccgtaaa gactggcgaa     360
   cagttcatac agagtctctt acgactcaat gacaagaaga aaatcttcgt caacatggtg     420
   gagcacgaca cacttgtcta ctccaaaaat atcaaagata cagtctcaga agaccaaagg     480
   gcaattgaga cttttcaaca aagggtaata tccggaaacc tcctcggatt ccattgccca     540
   gctatctgtc actttattgt gaagatagtg gaaaaggaag gtggctccta caaatgccat     600
   cattgcgata aaggaaaggc catcgttgaa gatgcctctg ccgacagtgg tcccaaagat     660
   ggacccccac ccacgaggag catcgtggaa aaagaagacg ttccaaccac gtcttcaaag     720
   caagtggatt gatgtgatat ctccactgac gtaagggatg acgcacaatc ccactatcct     780
   tcgcaagacc cttcctctat ataaggaagt tcatttcatt tggagagaac acg            833
<210> 8
   <211> 195
   <212> DNA
   <213> Artificial Sequence
<220>
   <223> sintetic
<220>
   <221> misc_feature
   <223> Human defensin with codon usage in citrus
<400> 8
   atgagagttc tttatcttct tttcagcttc cttttcattt tccttatgcc acttccaggt      60
   gttttcggtg gtattggtga tccagttaca tgccttaaga gcggtgctat ttgccatcca     120
   gttttctgcc caagaagata taagcaaatt ggtacatgcg gtcttccagg tacaagatgc     180
   tgcaagaaga agtag                                                      195
<210> 9
   <211> 72
   <212> DNA
   <213> Artificial Sequence
<220>
   <223> sintetic
<220>
   <221> misc_feature
   <223> Magainin with codon usage in citrus
<400> 9
   ggtattggta agtatcttca tagcgctaag aagtttggta aggcttgggt tggtgaaatt        60
   atgaatagct ag                                                            72
<210> 10
   <211> 192
   <212> DNA
   <213> Artificial Sequence
<220>
   <223> sintetic
<220>
   <221> misc_feature
   <223> Cecropin with codon usage in citrus
<400> 10
   atgaatttcg ctaagattct tagctttgtt ttcgctcttg ttcttgctct tagcatgaca        60
   agcgctgctc cagaaccaag atggaagatt ttcaagaaga ttgaaaagat gggtagaaat       120
   attagagatg gtattgttaa ggctggtcca gctattgaag ttcttggtag cgctaaggct       180
   attggtaagt ag                                                           192
<210> 11
   <211> 375
   <212> DNA
   <213> Artificial Sequence
<220>
   <223> sintetic
<220>
   <221> misc_feature
   <223> Sarcotoxin with codon usage in citrus
<400> 11
   atgaattttc aaaatatttt catttttgtt gctcttattc ttgctgtttt tgctggtcaa 60
   agccaagctg gttggcttaa gaagattggt aagaagattg aaagagttgg tcaacataca 120
   gatgctacaa ttcaaggtct tggtattgct caacaagctg ctaatgttgc tgctacagct 180
   agaggtatga attttcaaaa tattttcatt tttgttgctc ttattcttgc tgtttttgct 240
   ggtcaaagcc aagctggttg gcttaagaag attggtaaga agattgaaag agttggtcaa 300
   catacagatg ctacaattca aggtcttggt attgctcaac aagctgctaa tgttgctgct 360
   acagctagag gttag 375
<210> 12
   <211> 51
   <212> DNA
   <213> Artificial Sequence
<220>
   <223> sintetic
<220>
   <221> misc_feature
   <223> Indolicidin with codon usage in citrus
<400> 12
   atttgcttga agaagtggca atggtggcaa tggagaagat gcaagtatta g 51
<210> 13
   <211> 1151
   <212> DNA
   <213> Arabidopsis thaliana
<220>
   <221> misc_feature
   <223> Phloem specific expression promotor 59880 of Arabidopsis thaliana
<400> 13
   ttctcctaat catacgttat tggaaacatt aacgcggttt gctttatgca actttgaaac 60
   atttgctaat ttgttgatat aaaatttggc tatttatact gtttagttta gtaaggaatt 120
   aacacaaatg ttttagcgca ttattgcaca tttgtgtgaa ataatatact attttcggga 180
   aaatcataat cgaaatagaa ttccaaaatt tccacgtggc atattatgaa aggaatattc 240
   cagctcgggt aagctggcct ccatcaccgg ttatgatgaa aggtgagccg aaagtggagc 300
   caacctgaga tgccccgcat gagagccata tgcttcttcg acctttcatt ctatattctt 360
   tccttttaaa gatagcaact attgatcaaa aaaaagaaga aaaaagatag gaactataga 420
   actatagtct ttttttttca acgaagaaca aatatatggt tatgaactat tcttaatgga 480
   tggtcaaaca gattttttga tttttttcta agaaattttg catgtgaata cataactaca 540
   gttaattcca ttcaactgtt acttttcttt gggtgaaaca ataggttggc aacttggcat 600
   agatatcaac atcagagttt tgaacaaatc tatcatcatt taagaaatga tagaaaatca 660
   agtattgttt ttcaaatcaa gaaatatcaa agaagaattc tgtattaagt ttagttctca 720
   tggaagccat agaatatatc agaagtttga aacaaacagc aaatatatgg aataaaaaac 780
   atagttattg gaataaatag aatagttatt tttttttgtt tggcataaaa taaagaaaat 840
   tatttgttca acaaattgtt acagatttca cttatgtata ttttgaacta attcttattt 900
   ttatattaat taaatcaaga ttttactttt gaaaaataat taatgaattt atgaaacaat 960
   ctctatgtaa ataaaataaa gcaaattaaa acgctcacac atgtttactt attcggacta 1020
   gagagcttcc gcataaagct gaggaaaaaa aagagagaag acgcacaccg aagaaaacac 1080
   acaagactcc attattctcc tgcttcttcg tctcttcaat tattttctct gagactagct 1140
   ttgattcaac c 1151
<210> 14
   <211> 30
   <212> DNA
   <213> Artificial Sequence
<220>
   <223> sintetis
<220>
   <221> misc_feature
   <223> Forward oligonucleotide for lysozyme gene amplification with codon usage in citrus
<400> 14
   aggttttcga aagatgcgaa cttgctagaa 30
<210> 15
   <211> 30
   <212> DNA
   <213> Artificial Sequence
<220>
   <223> sintetic
<220>
   <221> misc_feature
   <223> Reverse oligonucleotide for lysozyme gene amplification with codon usage in citrus
<400> 15
   aaacaccgca accttgaaca tattgtctgc 30
<210> 16
   <211> 30
   <212> DNA
   <213> Artificial Sequence
<220>
   <223> sintetic
<220>
   <221> misc_feature
   <223> Forward oligonucleotide for defensin gene amplification with codon usage in citrus
<400> 16
   atgagagttc tttatcttct tttcagcttc 30
<210> 17
   <211> 30
   <212> DNA
   <213> Artificial Sequence
<220>
   <223> sintetic
<220>
   <221> misc_feature
   <223> Reverse oligonucleotide for defensin gene amplification with codon usage in citrus
<400> 17
   acttcttctt gcagcatctt gtacctggaa 30